# EUROPEAN PATENT APPLICATION

(11) **EP 4 241 714 A2**
(43) Date of publication of application: **13.09.2023**
(21) Application number: 23182014.3
(22) Date of filing: 15.09.2017
(51) Int. Cl.: A61B 18/14

(54) **METHODS AND APPARATUS FOR ELECTROSURGICAL ILLUMINATION**

(30) Priority: 16.09.2016 US 201662395529 P
(62) Divisional of application: 17851581.3
(71) Applicant: Invuity, Inc., San Francisco CA 94107 (US)
(72) Inventor: VAYSER, Alex, Mission Viejo, CA, 92692 (US)
(74) Representative: Röthinger, Rainer

(57) **Abstract**

An illuminated electrosurgical instrument (1002) comprises: a handle (12; 204; 1004; 1302; 1502; 2304) with a proximal portion and a distal portion, an electrosurgical tip (1802; 2114; 2214) extending distally of the handle (12; 204; 1004; 1302; 1502; 2304), wherein the electrosurgical tip (1802; 2114; 2214) is configured to perform electrosurgery using a radiofrequency (RF) energy, and an optical waveguide (202; 702; 1006; 1202; 1304; 1402; 15101; 1808; 2302) that extends continuously and at least partially circumferentially about the electrosurgical tip (1802; 2114; 2214), wherein the optical waveguide (202; 702; 1006; 1202; 1304; 1402; 15101; 1808; 2302) has a proximal end and a distal end, a plurality of light sources (216; 306, 324; 416, 422; 606, 652; 706; 1110; 1410; 1606; 1828; 2116) optically coupled to the proximal end of the optical waveguide (202; 702; 1006; 1202; 1304; 1402; 15101; 1808; 2302), wherein the plurality of light sources (216; 306, 324; 416, 422; 606, 652; 706; 1110; 1410; 1606; 1828; 2116) is configured to generate light at a plurality of colors, and an illumination switch (206) that is configured to control the plurality of light sources (216; 306, 324; 416, 422; 606, 652; 706; 1110; 1410; 1606; 1828; 2116), wherein the illumination switch (206) comprises a slidable switch that is configured to control at least one of an intensity of the light or a color of the light based on how far the slidable switch has been slid, and wherein the optical waveguide (202; 702; 1006; 1202; 1304; 1402; 15101; 1808; 2302) is configured to transmit the light from the plurality of light sources (216; 306, 324; 416, 422; 606, 652; 706; 1110; 1410; 1606; 1828; 2116).

## Description

### CROSS-REFERENCE TO RELATED APPLICATIONS

The present application claims priority to U.S. Provisional Patent Application No. 62/395,529, filed on September 16, 2016, which is herein incorporated by reference in its entirety.

The present application is related to US Patent Application No. 14/962,942 (Attorney Docket No. 40556-740.201) filed December 8, 2015; the entire contents of which are incorporated herein by reference.

### BACKGROUND OF THE INVENTION

1. Field of the Invention. The present application generally relates to medical devices, systems and methods, and more particularly relates to illuminated electrosurgical instruments. Conventional electrosurgical tools are commonly used in most surgical procedures. Energy hand-pieces generally include a hand-piece (also referred to herein as a handle) and an energy tip. The hand-piece is ergonomically shaped to allow a surgeon to manipulate the hand-piece during surgery and position the energy tip into a desired position where energy, typically radiofrequency (RF) energy is delivered to target tissue to cut or coagulate the tissue. One of the potential challenges with these devices is their use in deep, dark openings that are difficult to access without obstructing the surgical field, and which are difficult to adequately illuminate. Commercially available energy hand-pieces do not always include a light source for illuminating the surgical field and thus lighting must be supplied by another device such as a headlamp the surgeon wears or an overhead light that is manually adjusted, each of which have their own limitations under certain conditions. The hand-pieces that do provide illumination may have illumination elements such as light emitting diodes (LEDs) that are mounted releasably or fixedly into the handle of the device, but this is not necessarily the optimal position or distance from the work surface or target, and these devices may not have optimized lensing for collecting and shaping the light, and advanced light shaping may require larger profile lenses that are not practical for a surgical application with limited profile. Lenses may also add significant cost to the product, which is often a single use device that is discarded after a procedure is performed. Light shaping is also critical as conventional LED dies have broad Lambertian outputs that require collection and directionality. High powered LEDs also generate significant heat from the LED die and the heat may be conducted to the core of the LED board. Therefore, cooling is required to keep the entire device safe, especially when in contact with a patient. Also, it would be desirable to keep the light as close to the surgical target as possible thereby ensuring sufficient brightness and intensity. Many commercially available devices have LEDs positioned at the very distal tip of the device but this can result in challenges with lighting quality such as sufficient brightness, device profile, beam directionality, light shaping, and thermal management. Moreover, many illuminated hand-pieces produce unwanted shadows or reflections from the energy tip. Therefore, the light provided by the LEDs is preferably thermally safe, low profile, and directed and shaped for optimal illumination of the surgical target. It would therefore be desirable to provide improved energy hand-pieces that provide better lighting in order to illuminate a work surface or target area such as a surgical field. At least some of these objectives will be met by the embodiments disclosed below.

### SUMMARY OF THE INVENTION

The present invention generally relates to medical systems, devices and methods, and more particularly relates to illuminated energy devices, systems and methods.

In an aspect of the present disclosure, an illuminated electrosurgical instrument comprises a handle with a proximal portion and a distal portion, an illumination element coupled to the handle near the distal portion thereof, and an electrosurgical tip coupled to the illumination element. The illumination element may extend continuously and at least partially circumferentially about the electrosurgical tip. The illumination element may cast a beam of light that is continuous and at least partially annular distal to the illumination element and either (1) proximal to a distal tip of the electrosurgical tip, (2) at the distal tip of the electrosurgical tip, or (3) distal to the distal tip of the electrosurgical tip. The illumination element may deliver a continuous, annular beam of light to a target. The illumination element may comprise an optical waveguide, an organic light emitting diode (OLED), one or more discrete light emitting diodes (LEDs), or a plurality of optic fibers. The cross-sectional shape of the illumination element may take on several forms including those selected from one of the following: a partial or complete circle, a partial or complete oval, a partial or complete ellipse, a partial or complete square, a partial or complete rectangle, and a partial or complete polygon. The illumination element may be adjustably coupled to the handle such that actuation of the illumination element in a first direction moves the illumination element toward the proximal portion of the handle and actuation of the illumination element in a second direction opposite the first direction moves the illumination element toward the distal portion of the handle and the electrosurgical tip may also be adjustably coupled to the illumination element such that actuation of the electrosurgical tip in a first direction moves the electrosurgical tip toward the proximal portion of the handle actuation of the electrosurgical tip in a second direction opposite the first direction moves the electrosurgical tip toward the distal portion of the handle. Furthermore, the electrosurgical tip may alone be adjustably coupled to the illumination element such that actuation of the electrosurgical tip in a first direction moves the electrosurgical tip toward the proximal portion of the handle and actuation of the electrosurgical tip in a second direction opposite the first direction moves the electrosurgical tip toward the distal portion of the handle. The electrosurgical tip may be removably coupled to the illumination element. A light source may be coupled to a proximal end of the illumination element and a battery may be disposed within the handle to supply power to the light source.

In another aspect of the present disclosure, a method for illuminating a surgical target, comprises: providing an electrosurgical tip having a circumferential illumination element; illuminating the surgical target with light from the illumination element; and moving the illumination element toward or away from the surgical target, thereby adjusting the illumination on the surgical target. The method may further comprise replacing the electrosurgical tip with a different electrosurgical tip and locking one or more of the electrosurgical tip or the illumination element after illumination adjustment.

In another aspect of the present disclosure, an illuminated electrosurgical instrument comprises a handle, an illumination element coupled to the handle, an electrosurgical tip coupled to the illumination element, and an optical element (to deliver a continuous, at least partially annular beam of light to a target) disposed at least partially and circumferentially around the electrosurgical tip and is coupled to the illumination element. The illumination element may comprise an organic light emitting diode (OLED), one or more discrete light emitting diodes (LEDs), or a plurality of optic fibers. The illumination element may be adjustably coupled to the handle such that actuation of the illumination element in a first direction moves the illumination element toward the proximal portion of the handle and actuation of the illumination element in a second direction opposite the first direction moves the illumination element toward the distal portion of the handle and the electrosurgical tip may also be adjustably coupled to the illumination element such that actuation of the electrosurgical tip in a first direction moves the electrosurgical tip toward the proximal portion of the handle actuation of the electrosurgical tip in a second direction opposite the first direction moves the electrosurgical tip toward the distal portion of the handle. Furthermore, the electrosurgical tip may alone be adjustably coupled to the illumination element such that actuation of the electrosurgical tip in a first direction moves the electrosurgical tip toward the proximal portion of the handle and actuation of the electrosurgical tip in a second direction opposite the first direction moves the electrosurgical tip toward the distal portion of the handle. The electrosurgical tip may be removably coupled to the illumination element. A light source may be coupled to a proximal end of the illumination element and a battery may be disposed within the handle to supply power to the light source. The optical element comprises one or more of a lens, a hollow reflector, a gradient lens, a lenslet, a plurality of lenslets, a filter, or a coating for desired optical properties. The optical element may be concentric to the electrosurgical tip.

In another aspect of the present disclosure, a method for illuminating a surgical target comprises: providing an electrosurgical device having an optical element disposed at least partially and circumferentially around an electrosurgical tip and illuminating the surgical target with light from the optical element. The method may further comprise moving the optic fiber toward or away from the surgical target, thereby adjusting the illumination on the surgical target.

In another aspect of the present disclosure, an illuminated electrosurgical instrument comprises a handle with a proximal portion and a distal portion, an electrosurgical tip coupled to the handle near the distal portion thereof and an optic fiber (or plurality of optic fibers) with a proximal portion and a distal portion, the distal portion of the optic fiber coupled to the proximal portion of the handle, wherein light is delivered by the optic fiber to a target. The optic fiber may extend proximally outside the proximal portion of the handle and the proximal portion of the optic fiber is coupled to a light source (such as an LED, a plurality of LEDs, a laser, a xenon lamp, or any combination thereof). The illumination element may be adjustably coupled to the handle such that actuation of the illumination element in a first direction moves the illumination element toward the proximal portion of the handle and actuation of the illumination element in a second direction opposite the first direction moves the illumination element toward the distal portion of the handle and the electrosurgical tip may also be adjustably coupled to the illumination element such that actuation of the electrosurgical tip in a first direction moves the electrosurgical tip toward the proximal portion of the handle actuation of the electrosurgical tip in a second direction opposite the first direction moves the electrosurgical tip toward the distal portion of the handle. Furthermore, the electrosurgical tip may alone be adjustably coupled to the illumination element such that actuation of the electrosurgical tip in a first direction moves the electrosurgical tip toward the proximal portion of the handle and actuation of the electrosurgical tip in a second direction opposite the first direction moves the electrosurgical tip toward the distal portion of the handle.

In another aspect of the present disclosure, a method for illuminating a surgical target comprises: providing an electrosurgical device having a handle and an optic fiber, at least a portion of the optic fiber disposed within the handle, the fiber optic coupled to a light source and illuminating the surgical target with light from the optic fiber. The optic fiber may be moved toward or away from the surgical target to adjust the illumination on the surgical target.

In another aspect of the present disclosure, an illuminated electrosurgical instrument comprises a handle with a proximal end and a distal end, an electrosurgical tip coupled to the handle near the distal end thereof, a first illumination element (with a proximal end and a distal end) disposed continuously and at least partially circumferentially around the electrosurgical tip delivering a first continuous, annular beam of light extending around the electrosurgical tip to a target, and a second illumination element (with a proximal end and a distal end) disposed on the handle near the distal end thereof delivering a second light to a target. The first illumination element may comprise an optical waveguide, one or more LEDs, an OLED, a plurality of optic fibers, or any combination thereof. The second illumination element may comprise an optical waveguide, one or more LEDs, an OLED, one or more optic fibers, or any combination thereof. The first light for the first illumination element may be provided by a first light source disposed within the handle or an external light source. The external light source may be an LED, a plurality of LEDs, a laser, a xenon lamp, or any combination thereof. The second light for the second element may be provided by a second light source disposed within the handle or an external light source. The external light source may be an LED, a plurality of LEDs, a laser, a xenon lamp, or any combination thereof. The first illumination element and the second illumination element may be concentric. The illumination element may be adjustably coupled to the handle such that actuation of the illumination element in a first direction moves the illumination element toward the proximal portion of the handle and actuation of the illumination element in a second direction opposite the first direction moves the illumination element toward the distal portion of the handle and the electrosurgical tip may also be adjustably coupled to the illumination element such that actuation of the electrosurgical tip in a first direction moves the electrosurgical tip toward the proximal portion of the handle actuation of the electrosurgical tip in a second direction opposite the first direction moves the electrosurgical tip toward the distal portion of the handle. Furthermore, the electrosurgical tip may alone be adjustably coupled to the illumination element such that actuation of the electrosurgical tip in a first direction moves the electrosurgical tip toward the proximal portion of the handle and actuation of the electrosurgical tip in a second direction opposite the first direction moves the electrosurgical tip toward the distal portion of the handle.

In another aspect of the present disclosure, a method for illuminating a surgical target comprises: providing an electrosurgical device having a first illumination element and a second illumination element; illuminating the surgical target with light from the first illumination element; and illuminating the surgical target with light from the second illumination element.Moving the first illumination element toward or away from the surgical target may adjust the illumination on the surgical target.

In yet another aspect, an illuminated electrosurgical instrument comprises a handle, an illumination element and an electrosurgical tip. The handle has a proximal portion and a distal portion. The illumination element is coupled to the handle near the distal portion of the handle, and the electrosurgical tip is coupled to the illumination element. The illumination element extends continuously and circumferentially about the electrosurgical tip. The illumination element comprises a slot disposed at least partially through a thickness of the illumination element, and the slot extends axially and at least partially along a length of the illumination element. At least a portion of the electrosurgical tip is disposed in the slot.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figs. 1A-1D illustrate standard illuminated energy hand-pieces.
Figs. 2A-2G illustrate an energy hand-piece with a circumferential illumination element.
Figs. 3A-3B illustrate exemplary embodiments of an optical waveguide.
Figs. 4A-4F illustrate exemplary embodiments of illumination elements.
Figs. 5A-5D illustrate exemplary embodiments of a conductor element adjacent the optical waveguide.
Figs. 6A-6D illustrate an exemplary embodiment of a light source comprising LEDs.
Fig. 6E illustrates another exemplary embodiment of an electrode with an illumination element.
Fig. 7 illustrates an exemplary embodiment of an optical waveguide with an electrode.
Fig. 8 highlights the proximal portion of the waveguide in Fig. 7.
Fig. 9 illustrates an exemplary embodiment of an illuminated electrosurgical device with channels.
Fig. 10 illustrates an exemplary embodiment of an illuminated hand-piece with energy tip.
Figs. 11-12 illustrate cross-sections of exemplary embodiments of illuminated hand-pieces with an energy tip.
Figs. 13A-13B illustrate exemplary embodiments of an illumination element coupled to an energy tip or conductor element.
Fig. 13C illustrates a coating on the electrode.
Figs. 14A-14C illustrate alternative positions of a light source relative to an illumination element.
Fig. 15 illustrates an exemplary embodiment of a locking mechanism.
Figs. 16A-16D illustrate another exemplary embodiment of an illuminated electrosurgical device.
Figs. 17A-17D illustrate an optional battery feature.
Figs. 18A-18F illustrate another exemplary embodiment of an illuminated electrosurgical device.
Figs. 19A-19D show various electrode cross-sections.
Figs. 20A-20F illustrate exemplary embodiments of light cast from an illumination element.
Figs. 21A-21F illustrate exemplary illuminated electrosurgical devices emphasizing a fiber optic feature.
Figs. 22A-22F illustrate exemplary illumination elements that are continuous and at least partially circumferential about a surgical instrument.
Fig. 23 is a schematic diagram illustrating operation of a surgical instrument with a movable shroud surrounding the waveguide.

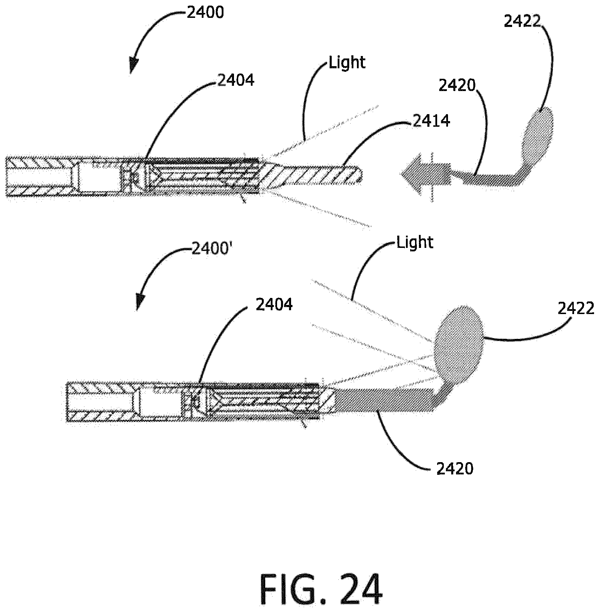

Fig. 24 is a schematic diagram illustrating operation of a surgical instrument in which a mirror attachment may be slipped over the electrosurgical tip.

### DETAILED DESCRIPTION OF THE INVENTION

Specific embodiments of the disclosed device, delivery system, and method will now be described with reference to the drawings. Nothing in this detailed description is intended to imply that any particular component, feature, or step is essential to the invention.

The present invention will be described in relation to illuminated energy hand-pieces used for example, during electrosurgery for cutting or coagulation of tissue. However, one of skill in the art will appreciate that this is not intended to be limiting and the devices and methods disclosed herein may be used with other instruments, and methods.

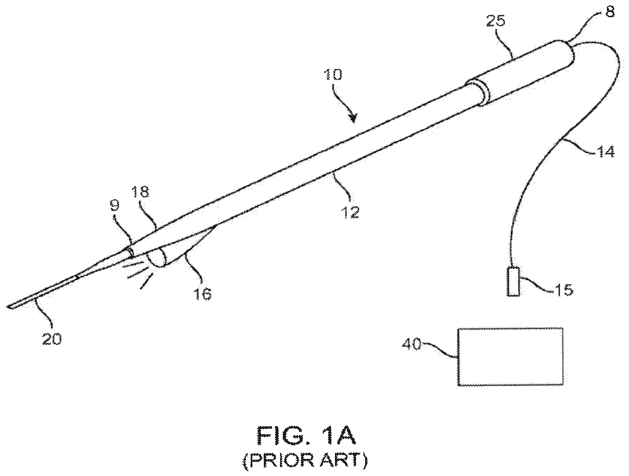

Fig. 1A illustrates a standard illuminated energy hand-piece 10 which includes a handle 12, an energy tip or electrode 20, an illumination element 16, a cable 14 and an external power source 40 that may be operably coupled with the cable 14. The external power source 40 may be used to provide energy such as RF energy to the electrode 20. Generally standard illumination energy devices have encapsulated power sources such as batteries in the handle or an external power source with a separate plug or connection. Because the illumination element is attached to a distal portion of the handle 12, light emitted from the illumination element 16 may not always have the desired intensity, directionality or uniformity or other desired optical properties when directed onto the surgical field. This may further be seen when different lengths of electrodes 20 are used with the handle 12 which would change the relative distance from the light source to the target, such as a surgical target. Since the intensity of light is inversely proportional to the square of the distance to the target, keeping the source as close to the target is desirable. Lenses may be used in conjunction with the illumination element 16, but these do not always provide the desired quality of light, especially since larger profile lenses are needed but these larger sizes are not always practical for a surgical application where space is very limited.

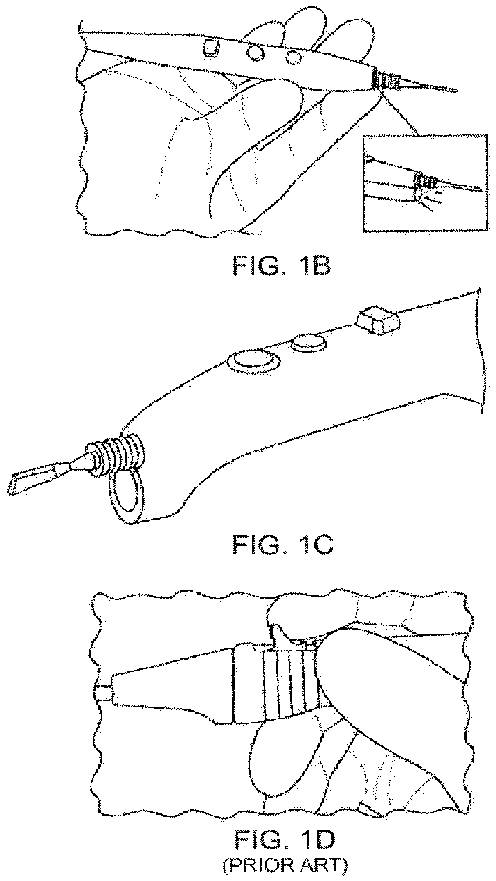

Figs. 1B-1D illustrate exemplary illuminated electrosurgical instruments. Fig. 1B illustrates an electrosurgical pencil having an RF electrode and an LED illumination element. Fig. 1C highlights the tip of the device in Fig. 1B. Because the LED is attached to the pencil, if a long electrosurgical tip is used, the LED may be too far away from the surgical field to adequately illuminate the tissue in the surgical field. Fig. 1D illustrates another electrosurgical pencil having an illumination source disposed in the pencil of the instrument, thereby resulting in a large profile of the device which can obstruct access to the surgical field.

Some of the challenges mentioned above may be overcome with the exemplary embodiments of illuminated electrosurgical instrument described below.

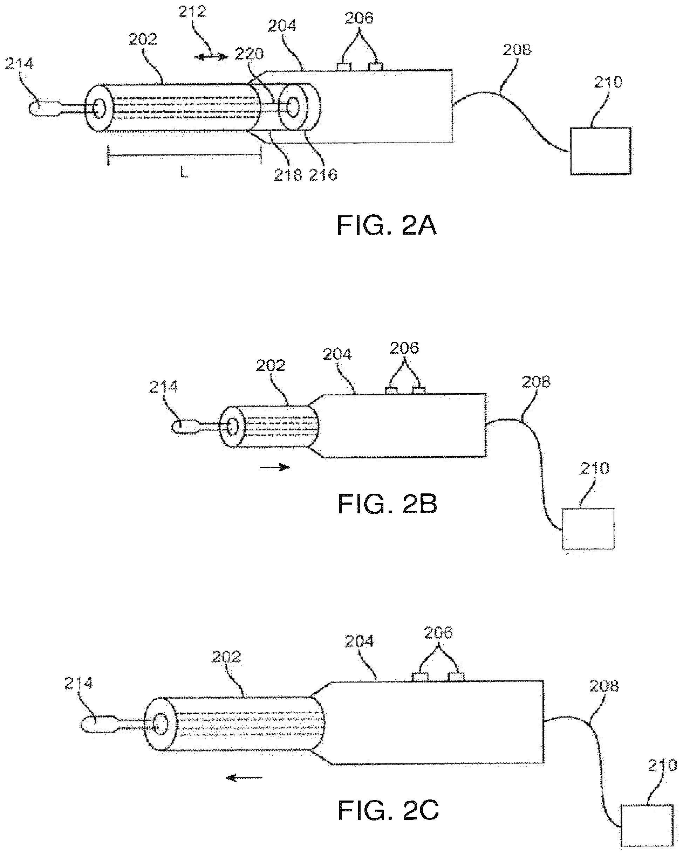

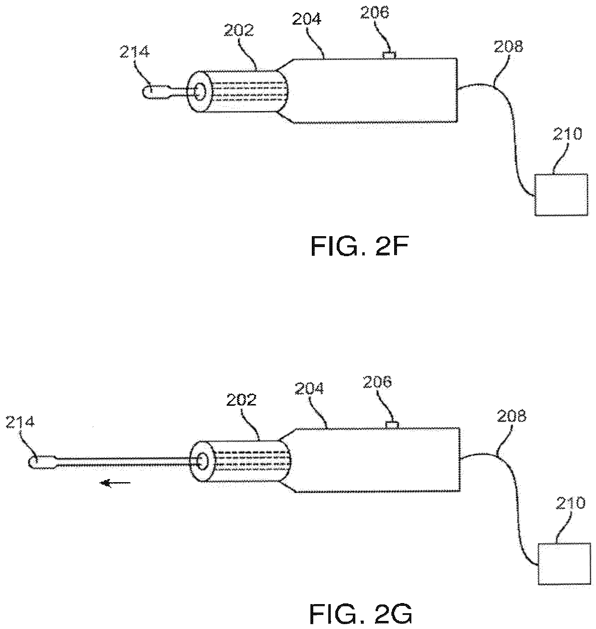

Fig. 2A illustrates an exemplary embodiment of an electrosurgical pencil having a circumferential illumination element 202, in this case an optical waveguide, coupled to a distal portion of a handle 204, and an electrode 214 (also referred to as an energy tip) for delivering energy, typically RF energy, to a tissue for coagulation or cutting, extending distally from the optical waveguide 202.

The waveguide 202 may be partially or fully circumferential around the energy tip 214. A cable 208 may be coupled to the proximal portion of the handle and this may operatively couple the energy hand-piece to an external power supply 210, such as an electrosurgical generator. The power supply 210 may provide RF energy to the electrode 214 through a conductor element 220, and may also provide power to a light source 216 which delivers light to the waveguide 202 through a proximal portion of the waveguide 218. Optionally, the power source 210 may also include an external light source (e.g. a xenon lamp, a laser, etc.) which can deliver light via a fiber optic cable included in cable 208 to introduce light into waveguide. The optional light source may be integral with the power source 210 or it may be a separate component.

The electrode 214 may be fixedly attached to the waveguide 202 or the handle 204, or it may be detachably connected thereto, which allows a user to replace electrode tips depending on the procedure being performed.

In an alternative embodiment a portion of the handle 204 may be integrated with micro LED die, thereby allowing the electrosurgical electrode tip 214 to provide power to generate light when the tip is inserted into the pencil because as current is activated, current also flows to the LED.

The optical waveguide 202 may be fixedly attached to the handle 204 or it may be adjustably attached thereto, such as with a movable connection to allow the length of the optical waveguide 202 to be adjusted based on the length of the electrode 214. Any mechanism known in the art may be used to allow adjustment of the movable optical waveguide 202, such as a collet, a threaded connection, a pin and detent mechanism, a spring loaded mechanism, a ratchet and pawl mechanism, etc. The light source 216 may be disposed in the handle 204 or coupled to a distal portion of the handle 204, or coupled to the proximal end of the waveguide 202, and may supply light to the optical waveguide. Thus in this or any embodiment, the light source 216 may move with the waveguide 202, and the waveguide 202 may move independently of the electrode 214. Any number of configurations of this device are possible, as described within the body of this specification. The energy tip 214 may therefore be fixedly connected to the waveguide 202 and the tip 214 may move together with the waveguide 202 as it is slid or otherwise moved inward or outward, or the tip 214 may be detachably connected to the waveguide 202 or the tip 214 may also move with the waveguide 202 as it is moved inward or outward. In still other embodiments, the tip 214 may be coupled to the handle 204, and the tip 214 may remain stationary as the waveguide 202 is moved, or the tip 214 may be moved independently of the waveguide 202.

The illumination element of any embodiment may be partially or fully circumferential about the electrode of any embodiment. The illumination element of any embodiment may be a hollow tubular waveguide having a central channel extending through the tube, and with the electrode extending partially or all the way through the central channel or the illumination element may be a solid rod with no space between the electrode and conductor wire and the inner surface of the optical waveguide. The illumination element of any embodiment may provide light that extends continuously and circumferentially about an electrode, as distinct from discrete light sources that may provide light that is discrete about or near an electrode. In whatever embodiment, the optical waveguide may be fixed or adjustable. When the optical waveguide is fixed, it has a specific tube length that is attached to the handle.

The light transmitted to a target region of any embodiment may comprise visible light comprising one or more wavelengths from about 390 nanometers to about 700 nanometers, preferably a bright white light. The light transmitted to a target region of any embodiment may comprise infrared, or near infrared, light comprising one or more wavelengths generally from about 700 nanometers to about 1 millimeter and preferably from about 700 nanometers to about 1 micrometer. The light transmitted to a target region of any embodiment may comprise ultraviolet light comprising one or more wavelengths from about 100 nanometers to about 390 nanometers. Light may be provided by one or more light sources. In some embodiments, the light may comprise bright, white light. In some embodiments, the light may have a specular or speckle-based pattern. In some embodiments, the wavelength of light transmitted to a target region may change as a function of time, distance from the target region, mode of operation, or type of surgical procedure. One or more types of light may be provided simultaneously from a single light source and/or illumination element. One or more types of light may be provided simultaneously from one or more light sources and/or illumination elements. The one or more types of light of any embodiment may be delivered continuously or it may strobe. Strobing light may take on any pulse repetition frequency.

In some embodiments, the optical waveguide 202 may slidably or otherwise extend away from or toward the handle 204. Figs. 2B-2G illustrate this feature. In Fig. 2B the optical waveguide 202 is collapsed into the handle 204, and in Fig. 2C the optical waveguide 202 is extended outward away from the handle 204. The optical waveguide 202 may be a fixed length, but may collapse into the handle 204 so that the length of the exposed portion of the optical waveguide 202 decreases, or the optical waveguide 202 may extend away from the handle 204 so that the length of the exposed portion of the optical waveguide 202 increases. Various mechanisms for allowing the telescoping of the optical waveguide 202 have been disclosed previously or are otherwise known in the art. Allowing the optical waveguide 202 to be adjusted allows the user to bring the light closer to the work surface such as a surgical target, or the light may be moved away from the work surface. This may be advantageous when a surgeon uses various length energy tips with the handle so when a long tip is used, a longer optical waveguide is desired to ensure that the light is delivered close to the target tissue, and similarly, when a short tip is used, a shorter optical waveguide is preferred so that the tip of the waveguide is not too close to the work surface. Thus, a variable length optical waveguide allows a user to adjust length as required and to position the light output relative to the electrode tip.

As illustrated in Figs. 2B-2C, the electrode 214 may be coupled to the optical waveguide such that as the optical waveguide 202 telescopes - slidably or otherwise extends - towards or away from the handle 204, a distance between a portion of the electrode 214, for instance its distal most end, and a portion of the optical waveguide 202, for instance its distal most end, remains substantially constant. In any embodiment of a waveguide, the waveguide may be a solid or hollow cylindrical shape, as well as other shapes. The waveguide may also have a constant cross-section, or the waveguide may be tapered or flared in either the proximal to distal direction, or in the distal to proximal direction.

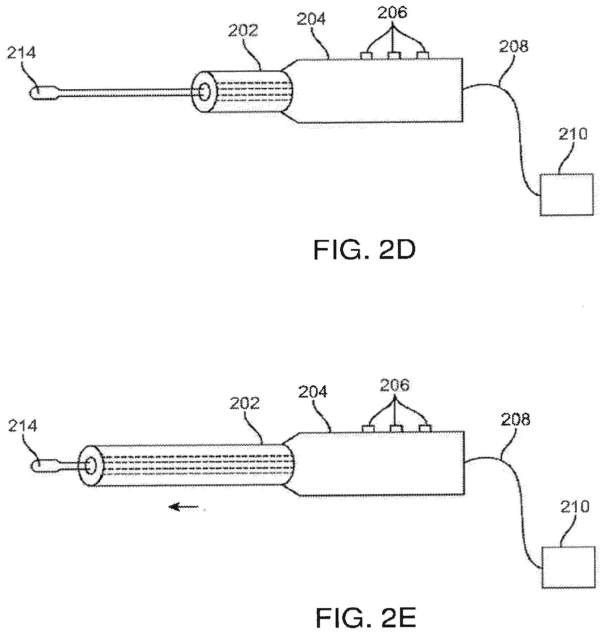

In some embodiments, such as that shown in Figs. 2D-2E, the optical waveguide 202 may telescope independently of the electrode 214. In some instances a distance from a portion of the electrode 214, for example its distal most end, and a portion of the handle 204, for example its distal most end, may remain substantially constant while the optical waveguide 202 telescopes from the handle 204. The optical waveguide 202 may telescope out from the handle 204 or it may telescope in toward the handle 204.

In some embodiments, such as that shown in Figs. 2F-2G, the electrode 214 may telescope independently of the optical waveguide 202. In some instances a distance from a portion of the optical waveguide 202, for example its distal most end, and a portion of the handle 204, for example its distal most end, may remain substantially constant while the electrode 214 telescopes from the handle 204. The electrode 214 may telescope out from the handle 204 or it may telescope in toward the handle 204.

The electrosurgical pencil may further comprise control switches 206 on the handle 204 that allow a user, surgeon, or operator to control the mode of operation from cutting or coagulation. The switches 206 of this or any embodiment may individually or collectively be of any type such as a joystick, pressure switch, proximity switch, push button switch, pushwheel switch, rocker switch, rotary switch, slide switch, speed switch, or toggle switch, or any combination thereof. The switches 206 may be biased or unbiased. Often two switches 206 are used, one for supplying RF current to the electrode that is optimal for cutting tissue, and the other switch supplies RF current to the electrode that is optimized for coagulating. These controls may also automatically provide light to the waveguide which then illuminates the surgical field when current is delivered from the electrode to tissue. In some embodiments, a separate illumination control switch may be disposed on the handle to active the light independently of the electrode power.

Some embodiments (such as those illustrated in Figs. 2D-2E) may use more than two switches 206 such as three switches, four switches, five switches, six switches, etc. In embodiments using more than two switches 206 one switch may make current available for the electrode to cut tissue, one switch may make current available to coagulate tissue, and one switch may cause the illumination element to illuminate a target region. The switches 206 of any embodiment may be disposed on the handle 204 in any position including along a linear, longitudinal axis on the surface of the handle 204, distributed circumferentially about the handle, on opposite sides of the handle 204, etc.

Some embodiments (such as those illustrated in Figs. 2F-2G) use a single switch 206 to control the electrosurgical pencil. For such embodiments and for all embodiments, the duration of switch engagement by a user or the pattern of switch engagement may control the action of the electrosurgical pencil. For example, if the switch were a pressable button, the user could press down quickly two times in succession to begin delivering current to the electrode suitable for coagulation and to begin illumination, a long press thereafter causing the current to become suitable for cutting, and another pair of quick presses may stop providing current to the electrode and turn off illumination. In another example, the illumination switch may be a slidable switch and the switches for cutting and coagulation may be pressable buttons. How far the slidable switch for illumination has been slid may control the intensity or the color or any property of the light delivered to the region such that sliding the switch a longer distance will produce brighter light than sliding the switch a short distance. Such examples are meant to be illustrative, not limiting, as one of skill in the art will appreciate that any combination of switches and any combination of switch engagement patterns or durations may be used to control any aspect of the electrosurgical pencil including but not limited to: delivering current to the electrode for cutting and/or coagulation of tissue; illuminating a target region; causing an illumination element, an electrosurgical tip, or both to extend from the body of the handle; activating a sensor; recording the result of a sensor; capturing a photograph; evacuating smoke, delivering fluid; removing fluid; or any other behavior specified herein.

Optionally, in this or any other embodiments disclosed herein, a single switch may be used to activate the electrosurgical pencil and a motion sensor, such as for example, an accelerometer, may be used to activate the light source instead of a mechanical switch. The motion sensor may operate as a switch. As long as motion is sensed, a signal generated by the motion sensor may be used to enable the power to keep the light on. When the electrosurgical sensor is not moving, the light is turned off. In some example embodiments, the motion sensor can also be used as a safety feature for energy at the tip. For example, if no motion of the pencil is sensed while the electrosurgical pencil is off, the power can be maintained in the off state if someone inadvertently activates the device, which may occur, for example, by tripping on a foot switch. For example, if no motion of the pencil is sensed while the electrosurgical pencil is off, the power can be maintained in the off state if someone inadvertently activates the device, which may occur, for example, by tripping on a foot switch.

The switches 206 of any embodiment may provide feedback in response to engagement. The feedback may be to inform the user that the device is in a certain operative state (for instance, delivering current to the electrode or illuminating the region) or to alert the user to a problem (such as not having enough power to drive current or illumination). Such feedback may be haptic feedback (such as from a vibration), audio feedback (such as a beep or tone), visual feedback (such as a light turning on or off), or any combination thereof. Visual feedback may be provided by the switches 206 themselves by, for instance, having a visual indicator, such as a light source, such as an LED, in the switch. The visual indicator may be disposed anywhere on the handle.

One or more indicators may be used with any embodiment described. The one or more indicators may be audio-based, visual-based, or haptic-based and may provide to the user feedback about the operative state or condition of the electrosurgical device, such as the current battery level, if a battery needs to be recharged, indicate the temperature of the device, temperature of the target region, etc. The indicator may be disposed anywhere on the handle or the switches. Audio-based indicators may provide a single beep, a drawn tone, a spoken message, a series of beeps, a whistle, or any combination of sounds to alert or inform the user. Visual-based indicators may provide one or more lights, constant light, flashing light, pulsing light, text on a display, numbers on a display, or any combination of visual cues to alert or inform the user. Haptic-based feedback may provide a constant vibration, pulsing vibrations, pulsating vibrations, or any combination thereof.

The features represented collectively across Figs. 2A-2F may be combined in any matter in any embodiment herein such that: the electrode 214 may telescope with the optical waveguide 202 (Figs. 2A-2B); the optical waveguide 202 may telescope independently of the electrode 214 (Figs. 2C-2D); the electrode 214 may telescope independently of the optical waveguide 202 (Figs. 2E-2F); and/or the electrode 214 and optical waveguide 202 do not telescope. Furthermore, the electrode 214 may telescope as a function of the telescoping of the optical waveguide 202, the function including but not limited to: the electrode 214 traversing a distance less than, equal to, or greater than a distance traveled by the optical waveguide 202 and/or the electrode 214 rotatably translating as a function of the distance traveled by the optical waveguide 202, such as turning clockwise or counter-clockwise. Similarly, the optical waveguide 202 may telescope as a function of the telescoping of the electrode 214, the function including but not limited to: the optical waveguide 202 traversing a distance less than, equal to, or greater than a distance traveled by the electrode 214; the optical waveguide 202 traversing a distance corresponding to a non-linear function of the distance traveled by the electrode 214, such as the square of the distance traveled by the electrode 214; and/or the optical waveguide 202 rotatably translating as a function of the distance traveled by electrode 214, such as turning clockwise or counter-clockwise. Any of the aforementioned translational relationships between distances traveled by either the electrode 214 or the optical waveguide 202, or both, may be combined in any of the embodiments disclosed herein.

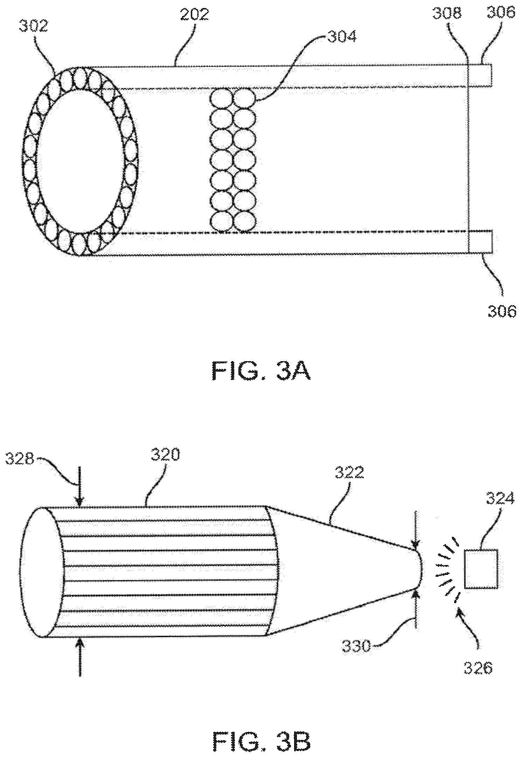

Figs. 3A-3B show that any embodiment of the illumination element 202 (in this case represented by an optical waveguide) may include optical structures such as lenslets 302 on the distal end of the tube, or the lenslets 302 may be disposed on an inner surface, an outer surface, or any distal portion 304 of the tube. The lenslets 302 help to extract and shape the light emitted from the waveguide 202. The proximal end of the waveguide 202 may include one or more light sources 306 which provide light to the waveguide 202. The light source 306 may be a single LED, one or more LEDs, an array of LEDs, a laser, a lamp, an incandescent light bulb, a compact fluorescence lamp, or a xenon lamp, or any combination thereof. The light elements 306 may be coupled to the waveguide 202 in any number of ways, including butt coupling to other coupling mechanisms, such as where the proximal end of the optical waveguide 202 has a parabolic shape to capture the broad divergence of light emitted from a light source 306. In this embodiment, the ratio of the size of the waveguide diameter to the light input 308 size may preferably be a minimum ratio of 2:1 as shown in Fig. 3A. Alternatively, the ratio may lie anywhere within the range from about 100:1 to about 1:1, preferably from about 30:1 to about 2:1, and more preferably about 5:1. The proximal portion of the waveguide may also have a parabolically shaped light input 322 with an input diameter 330 as shown in Fig. 3B having light source 324 emitting light 326 into waveguide 320. The body of the waveguide 202, 320 is preferably cylindrically shaped and may optionally have a plurality of facets along the outer circumference to provide multiple surfaces against which the light may bounce, thereby allowing the light to mix better along the waveguide. The body of the waveguide has an output diameter 328 through which the light passes and then is extracted. In preferred embodiments, the ratio of the output diameter 328 to the input diameter 330 is at least 2:1. Alternatively, the ratio may lie anywhere within the range from about 20:1 to about 2:1. Other embodiments have the light source 306, 324 positioned more distally located along the extended shaft, where the shaft may be composed of the waveguide, the light source, and a tube that provides heat sinking proximal to the light source. The tubular heat sink is described in more detail below. Additionally, the tube for heat sinking may be fabricated from any material that dissipates or preferentially transfers heat including but not limited to: aluminum alloys and pseudo alloys, copper alloys and pseudo alloys, diamond and diamond-based composites, magnesium alloys and pseudo alloys, and steel alloys and pseudo alloys, as well as other heat sink materials known in the art.

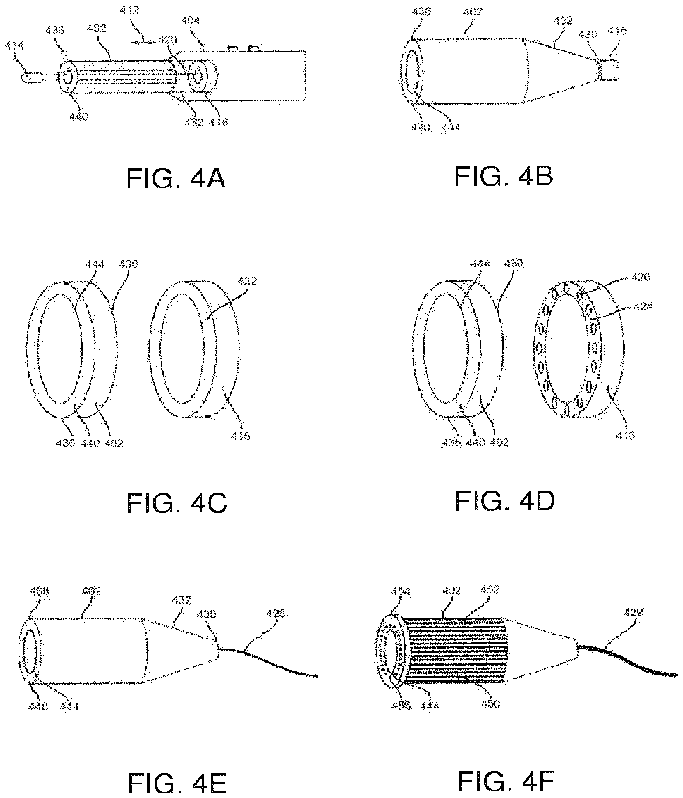

Figs. 4A-4F show exemplary embodiments of illumination elements. Some exemplary embodiments comprise an illumination element and a light source.

Fig. 4A shows an electrosurgical instrument comprising a hand-piece 404, a light source 416 disposed within the hand-piece 404, a moveable illumination element 402 coupled at its proximal end to the light source 416, and an electrode 414 coupled at the distal end 436 of the illumination element 402. Light is extracted from the illumination element 402 at its light extraction surface 440. The light extraction surface 440 may comprise light extraction structures including but not limited to grooves, prismatic lenses, lenslets, textured regions, polished regions, etc. The illumination element 402 may also have a proximal portion 432 configured to collect light from the light source 416. The proximal portion 432 may have a linear profile, a parabolic profile, or any profile described by the summation of one or more sine and/or cosine functions. The illumination element 402 may be an optical waveguide. The illumination element 402 may be slidably coupled to the hand-piece 404 or it may be fixed to the hand-piece 404.

Fig. 4B shows an exemplary embodiment of the combined pair of the light source 416 and the illumination element 402. The light source 416 may be a single source of light such as an LED or an array of LEDs. The illumination element 402 receives light from the light source 416 through the proximal end 430 of the optical waveguide. Shaping and/or guiding light through the illumination element 402 may be accomplished by using an optical waveguide as the illumination element 402, by using cladding disposed on one or more surfaces of the illumination element 402, or by having a proximal portion 432 shaped to direct light, or any combination thereof. The light extraction surface 440 may be such that the light extracted is focused or diffused or anywhere in the between. In some embodiments, the illumination element 402 has a channel 444 through which an electrode may pass.

Fig. 4C shows an exemplary embodiment of an illumination element 402 and a light source 416 comprising a continuous circumferential light source 422. The continuous light source may be an organic light emitting diode (OLED) that forms a partial or complete annulus or ring. The continuous light source of any embodiment may extend at least partially circumferential about an electrosurgical tip.

Fig. 4D shows an exemplary embodiment of an illumination element 402 and a light source 416 comprising a plurality of discrete light sources 426 arranged on a surface 424, the plurality of discrete light sources 426 disposed at least partially circumferentially. The discrete light sources 426 may each be arranged a distance (called here, a radial distance) from a central axis and spaced a distance (called here, a circumferential distance) from one another along a circumference traced by the radial distance. The radial distance - the distance from a defining point on an individual discrete light source to the central axis (for instance, a point on the discrete light source closest to the central axis, a point on the discrete light source farthest from the central axis, or the center of the discrete light source) - may be uniform about the central axis or it may be uneven. The radial distance may be about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 mm, or it may take on any value between any two aforementioned distances. The circumferential distance- the distance from a defining point on an individual discrete light source to a defining point on a neighboring individual discrete light source (for example, the shortest possible distance between any point on the discrete light source and its neighboring discrete light source, the farthest possible distance between any point on the discrete light source and its neighboring discrete light source, or the center of the discrete light source to the center of the neighboring discrete light source) - may be uniform about the circumference of it may be uneven. The circumferential distance may be about 0.1, 0.2, 0.3, 0.4, 0.5, 0.6, 0.7, 0.8, 0.9, , 2, 3, 4, 5, 6, 7, 8, 9, 10, 11, 12, 13, 14, 15, 16, 17, 18, 19, 20, 25, 30, 35, 40, 45, or 50 mm, or it may take on any value between any two aforementioned distances. The number of discrete light sources 426 may be about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 32, 34, 36, 38, 40, 42, 44, 46, 48, 50, 60, 70, 80, 90, 100, 200, 300, 500, 1,000, 2,000, 3,000, 5,000, 10,000 or it may take on any value between any two aforementioned numbers. Each of the discrete light sources 426 may be an LED, an OLED, or a fiber optic. The illumination element 402 may shape, form, guide, and/or combine the light delivered by the plurality of discrete light sources 426 such that a continuous circumferential beam of light forming a closed ring is emitted from the extraction surface 440 of the illumination element 402. The continuous beam of light may have a perimeter of constant or near constant light intensity. Alternatively, the continuous beam of light may have a perimeter of variable light intensity. When used optionally within any of the embodiments described herein, the continuous beam of light may illuminate a region around an energy tip, producing shadowless illumination of the region. The light extracted from the extraction surface 440 may be focused or diffuse.

Fig. 4E shows an exemplary embodiment of a fiber optic 428 coupled to the proximal end 430 of the illumination element 402, the fiber optic 428 delivering light from a light source (not illustrated). Though only a single fiber optic 428 is illustrated in Fig. 4E, one of skill in the art will appreciate that any number of fibers may be used.

Fig. 4F shows an exemplary embodiment of an illumination element comprised of a plurality of optic fibers 450 delivering light from a light source (not illustrated) to a target site through exposed distal ends 456 of the plurality of optic fibers 450. The exposed ends 456 of the plurality of optic fibers 450 may take on any pattern including: a random pattern wherein the spacing and/or orientation of the fibers does not follow a strict pattern, a radially symmetric pattern; an axially symmetric pattern; symmetric about any plane defined by the energy tip or the handle or both; spaced apart unevenly, and/or spaced equally apart from one another along a path defined by a fixed radius extending from the central axis of the illumination element, the electrode, the handle, or any combination thereof, the spacing between edges of the fiber optics preferably equal to or less than a distance equal to two times the diameter of an individual fiber optic from the plurality of optic fibers 450, more preferably equal to or less than a distance equal to the diameter of an individual fiber optic from the plurality of optic fibers 450, and even more preferably equal to or less than a distance equal to half the diameter of an individual fiber optic from the plurality of optic fibers 450.

The plurality of optic fibers 450 may be co-molded or assembled within a malleable material (such as silicone), and the fiber optic assembly 452 may then be formed so as to fit around an electrosurgical tip (such as any of those described herein). The plurality of fibers may have its distal end coincide with a support structure 454 (also referred to herein as a flange), designed to prevent the bending, stretching, torqueing, and/or dislodging of one or more of optic fibers from the plurality of optic fibers 450. The flange 454 may be made of the same malleable material as the fiber optic assembly 452 or it may be made of the same malleable material as the fiber optic assembly 452. that has been treated different to make it harder and/or stronger (including but not limited to chemical, mechanical, and thermal tempering such as treatment with an additional chemical, an extra peening procedure, or the application of further heating procedures, respectively). The flange 454 may optically guide light, such that the flange 454 directs the light delivered by the plurality of optic fibers 450 onto the target region. Different materials for the flange 454, such as aluminum, copper, magnesium, or steel may optionally be used, along with other materials known in the art. The flange 454 may comprise any illumination element 402 described herein and/or the flange 454 may be configured to receive any illumination element 402 described herein.

A fiber optic bundle 429 may deliver the plurality of optic fibers 450 to the illumination element.

In any of the embodiments, the light source may be disposed in a number of positions other than just at the proximal end of the illumination element. For example, the light source may be positioned between the proximal end and the distal end of the illumination element, or the light source may be positioned at the distal end. Additionally, the light source may be positioned in any number of orientations relative to the illumination element. Furthermore any illumination element described within this specification may be a combination of one or more light sources and one or more illumination elements as described herein.

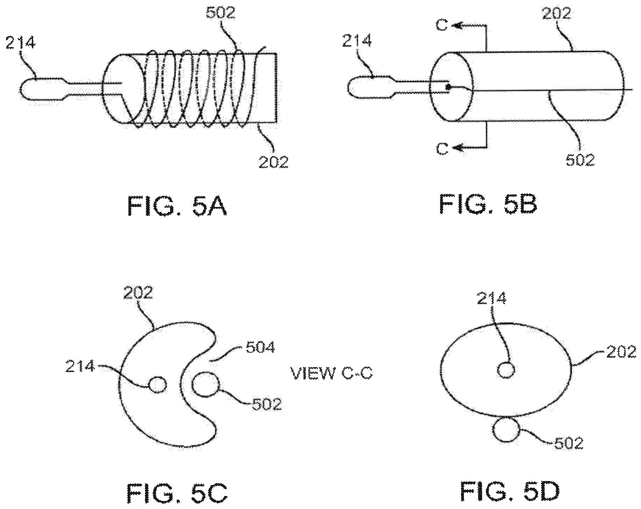

In some embodiments, the conductor element may pass through the illumination element (as shown in Fig. 2A), for instance, either a solid waveguide or a tubular waveguide, and provide energy from a power source (e.g. RF power supply) to the electrode. In other embodiments, such as in Fig. 5A, the conductor element may be a wire 502 that is wrapped helically or otherwise around an outside surface of the illumination element 202 and coupled to the electrode tip 214. The wire may have insulation to minimize any electromagnetic effects such as inductance created by the helical configuration. In Fig. 5B, the conductor element may be a wire 502 that runs preferably linearly along an outer surface of the waveguide. Fig. 5C shows an alternative embodiment of a cross-section taken along the line C-C in Fig. 5B where an optional recessed region 504 may be formed into the waveguide to accommodate the conductor element 502 to keep overall profile minimal. The recessed region 504 may be concave, angled, linear, or configured to closely correspond to the profile of the wire 502. In a variation of the embodiment in Fig. 5C, the conductor element may be shaped to complement the recessed region 504 of the waveguide so that when the conductor element and the waveguide are fit together, they form a cylinder having a circular cross-section. In still other embodiments, a tube (not illustrated) may be disposed around the waveguide similar to cladding disposed over the waveguide. The energy tip may be coupled to this outer tube, the outer tube configured to transfer heat from the region. The tube may be made of a metal (such as aluminum, copper, magnesium, or steel) or it may comprise a heat pipe. Fig. 5D illustrates still another embodiment of a conductor element 502 coupled with an illumination element 202. In this embodiment the conductor element 502 is coupled to an outer surface of the illumination element 202 and the conductor element 502 runs axially therealong. The resulting cross-section forms a figure eight-like shape with a large profile illumination element 202 and a smaller profile conductor element 502.

In any of the embodiments of the illumination element (which is preferably an optical waveguide), a coating or cladding may be applied thereto in order to provide desired optical properties to the illumination element, thereby enhancing its efficiency. The coating or cladding may be applied to an outside surface of the illumination element, to the central channel of the illumination element, or to an outer surface of the conducting element in order to optically isolate the conductor element from the illumination element as well as to provide electrical or other insulation as required. The layer of cladding also provides a physical barrier to prevent damage to the waveguide from scratching, abrasion or other damage caused by adjacent surgical instruments. Optionally, any embodiment described herein may use air gaps disposed adjacent the optical waveguide to enhance optical transmission of light through the illumination element by minimizing light loss, as well as by using standoffs to maintain an air gap between the illumination element and adjacent components.

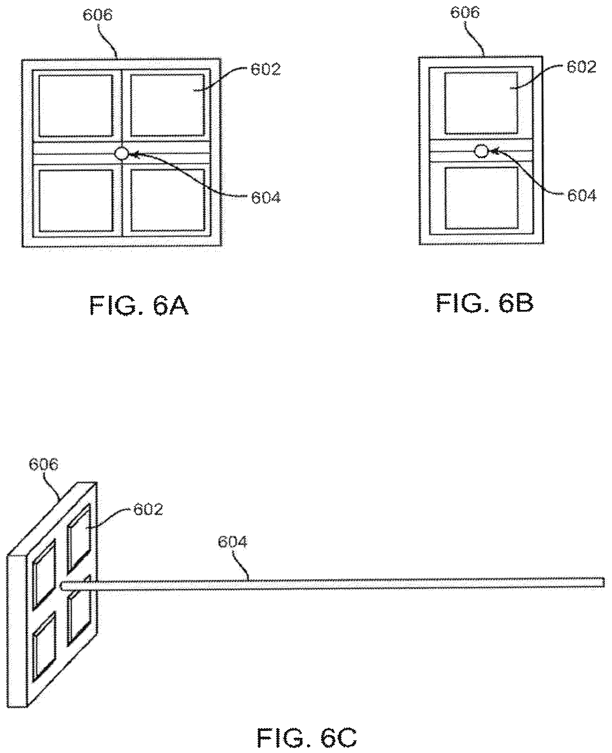

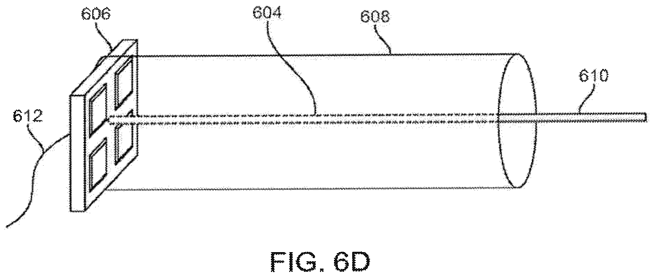

Figs. 6A-6D illustrate an exemplary embodiment of a light source comprising LEDs. In Fig. 6A the light source 606 includes an array of LEDs with die elements 602 formed into a square pattern. Any number or combination of die elements 602 may be used in order to provide the desired light. A conductor element 604 passes through the center of the light source 606 to transmit energy, power, and/or electricity from a power source to an energy tip. Fig. 6B illustrates an alternative light source 606 having an array of two LEDs with die elements 602 instead of the four LEDs illustrated in Fig. 6A. Any pattern and number of LEDs may be used. Fig. 6C illustrates the light source 606 with the conductor element 604 passing therethrough. Fig. 6D illustrates the light source 606 coupled to the proximal portion of the illumination element 608 with a power cable 612 coupled to the light source 606. The conductor element 604 extends axially through the illumination element with a distal portion 610 exposed so that it may be formed into an electrode tip or coupled with an electrode tip. Preferably the electrode tip is flat, and the conductor element may be round or flat in order to keep profile minimized. The illumination element 608 may be any of the embodiments of illumination elements described in this specification. It may be a round cylinder or it may have a hexagonal, octagonal, or other polygonal shaped cross-section for facilitating mixing of the light passing through the illumination element as discussed previously. The polygonal shaped cross-sections preferably have flat planar facets around the outer circumference of the illumination element. The flat surfaces enable better mixing of light from the light source so that the image of the actual LED die is not projected onto a target. The electrode tip may optionally be coupled directly to the light source. The proximal end of the illumination element may be parabola-shaped or have other custom shapes to provide better capture and mixing of light from the light source, the LEDs or any other light source. Any embodiment may optionally have a hole through the illumination element, the light source, or both, to accommodate the conducting element. The conducting element may fill the entire space in the waveguide and the two may be integral with one another. Moreover, the conducting element and the light source may be integrated onto a single circuit board.

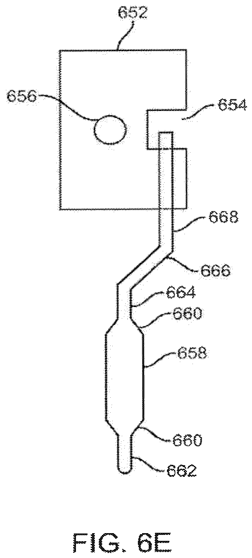

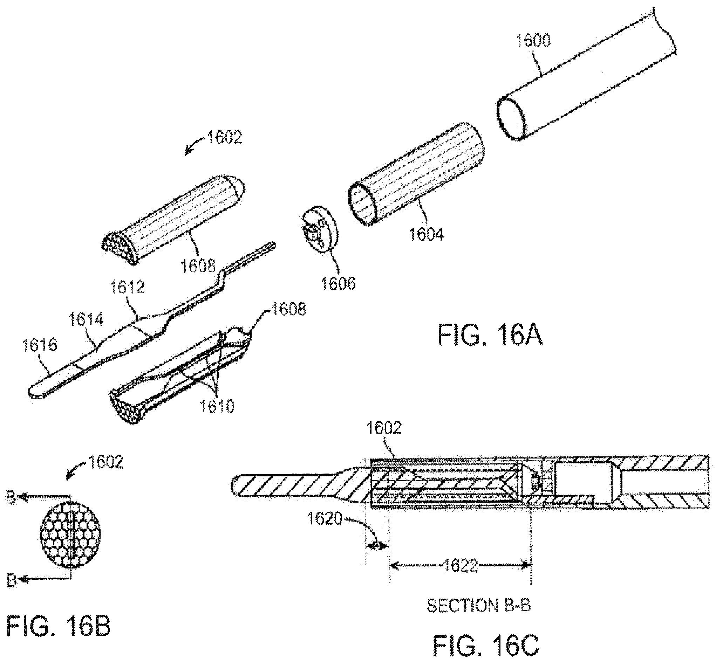

Fig. 6E illustrates an optional variation of the previous embodiment with the major difference being that only a single LED is used. The light source 652 includes a recessed or slotted region 654 which is sized and shaped to receive a portion of the conductor 668 which is connected to the electrode 658. A single LED 656 is disposed on the light source and may be centered thereon so as to be coaxial with the central axis of the electrode 658, and also, optionally, with that of the illumination element (not shown). The electrode 658 may have any of the features of any of the electrodes described herein including coatings or other insulation layers, especially those described with reference to Figs. 16A-16C. The electrode 658 includes a generally flat and planar section with proximal and distal tapered ends 660. The distal portion of the electrode forms an electrode tip 662 for delivering energy to tissue. The proximal portion forms an elongate arm 664 having an angled section 666 which couples the electrode to the conductor 668, thereby disposing the conductor off-center from the central axis of the electrode. While the embodiment of Fig. 6E illustrates a single LED, one of skill in the art will appreciate that any number of LEDs may be used, such as two, three, four, or more LEDs.

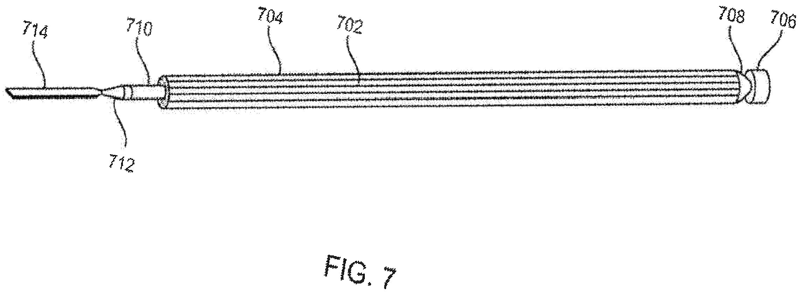

Fig. 7 illustrates an exemplary embodiment of an optical waveguide 702 with electrode tip 714. The electrode tip 714 may be a flat planar shape and may be coupled to a conductor element 712 which extends through the waveguide 702. A layer of cladding 710 may be disposed over the conductor element in order to isolate it from the waveguide 702. Additionally, a layer of cladding 704 may be disposed over the outer surface of the waveguide 702 to isolate it from blood or contaminants. The waveguide 702 in this embodiment is a polygonal shape (e.g. hexagonal, octagonal, etc.) having flat planar facets on the outer surface. The light source 706 (in this case, an LED, though any light source described in this specification may be used) 706 is coupled to the proximal end of the waveguide 702, and the proximal portion 708 of the waveguide 702 may parabolically shaped in order to receive a maximum amount of light from the LED 706. Other coupling means may be used to optically couple the LED 706 to the waveguide 702, such as by using lenses, hollow reflectors, gradient lenses, etc. Also, coatings may be applied to the waveguide 702 to enhance coupling efficiency. The light source 706 may be an LED or LED array, including any of the LED embodiments disclosed herein.

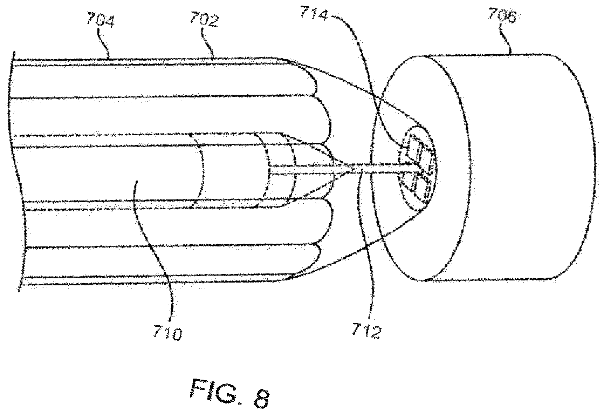

Fig. 8 illustrates the proximal portion of the waveguide 702 in Fig. 7. The conductor element 712 extends all the way through the waveguide 702 and exits the proximal-most end of the waveguide and is coupled with the illumination element 706. The conductor element 712 may be electrically coupled and/or bonded to the illumination element 706 or it may be disposed in a hole that extends through the illumination element 706. The illumination element in this embodiment is an array of LED elements 714 which generally takes the same form as described in Figs. 6A-6D. Additionally, the proximal portion of the waveguide may be parabolically shaped in order to capture a maximum amount of light from the LEDs. Cladding 710 may be disposed over the conductor element 712 to isolate the conductor element 712 from the waveguide 702 and this helps prevent light loss from contact between the two components. Also, as disclosed previously, air gaps may be used to help minimize light loss.

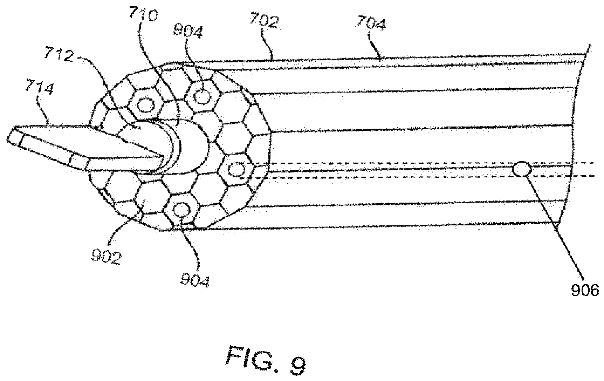

Any of the embodiments described herein may also include one or more channels that run through the illumination element. Fig. 9 illustrates an exemplary embodiment of an illuminated surgical instrument with channels 904 (also referred to as lumens), the channels 904 configured to either remove material from the region (such as smoke evacuation or fluid aspiration), to deliver material to the region (such as a fluid for cooling (e.g. water, saline, etc.) or a treatment fluid for treating the patient (the treatment fluid may comprise a drug, a medicine, an ointment, a clotting agent, etc), or the channel may house an additional medical instrument such as a camera or a sensor (e.g. a temperature sensor). The optical waveguide 702 includes cladding 704 disposed over the outer surface of the waveguide. Optical cladding may also line the transfer lumens to minimize light loss from the light source, particularly when it is a waveguide. The conductor element 712 extends through the waveguide and a layer of cladding 710 is disposed over the conductor element 712. The electrode tip 714 is coupled with the conductor element 712. The electrode may be bent relative to the conductor element 712 or the optical waveguide 702. Optional lenslets 902 on the distal face of the optical waveguide 712 may shape the light exiting the waveguide 712, thereby providing a desired illumination pattern on the target, here a surgical target. Channels 904 may extend axially all the way through the waveguide 712 to the proximal end thereof where the channels 904 may be coupled to a vacuum so that suction may be applied to the channels to draw out smoke or other excess material created during surgery, or a material delivery system so that material (such as a fluid for cooling or treating a patient) may be delivered to the region to help during surgery, or any combination thereof. The channels 904 may also house one or more sensors or medical instruments such as a camera, a temperature sensor, a sensor to measure the electrical properties of the region (such as impedance, conductance, etc.). The channels 904 of this or any embodiment may individually or collectively be configured to extend through the illumination element in many ways including partially or fully longitudinally down the illumination element, partially or fully circumferentially along the illumination element, partially or fully radially through the illumination element, or any combination thereof. In other embodiments where the optical waveguide 702 is a hollow tube, the central channel of the hollow tube may be used for material transfer. Optionally, in this or any other embodiments disclosed herein, a liquid filter 906 may be provided. For example, if channels or lumens are connected to a vacuum to suction smoke or other excess material during surgery, liquid filters may be added to prevent liquid from entering the lumens and thereby causing damage to internal electronics of the device. The filters may be configured to only pass air (or an unwanted gaseous material) and not liquid. The filter may be positioned at the distal end of the open lumen.

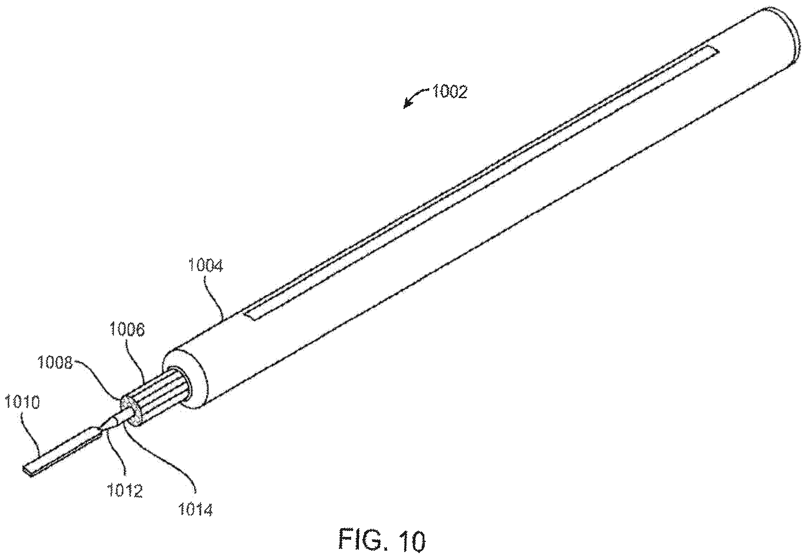

Fig. 10 illustrates another exemplary embodiment of an illuminated electrosurgical instrument 1002 demonstrating many of the individual features previously described above combined into a single exemplary embodiment. The illuminated electrosurgical instrument 1002 includes a handle 1004, an optical waveguide 1006, a conductor element 1012, and an energy tip 1010. The optical waveguide 1006 is preferably coaxially disposed in the handle 1004 and coaxial to the tip 1010 and may be either fixed to the handle or slidably adjustable as described above so that the exposed length of the waveguide 1006 may be increased or decreased as required. The waveguide 1006 preferably has a plurality of flat planar facets which form the polygonal outer surface of the waveguide, and this shape as discussed previously helps light mixing in the waveguide. An optional tube may be disposed over the waveguide 1006 and may be made from a heat transferring material (and preferably a heat conductive material) and acts as a heat sink to conduct heat away from the device. Additionally, optional lenslets 1008 may be disposed on the distal end of the optical waveguide 1006 to shape and direct the light so that the beam of light illuminates the surgical target properly. An optical cladding such as a polymer like fluorinated ethylene propylene (FEP), or a heat shrink, may be disposed over the waveguide 1006 to isolate it from direct contact with the handle 1004, thereby minimizing light leakage and protecting it from damage caused by contact with adjacent surgical instruments. A conductor element 1012 extends preferably coaxially through the optical waveguide 1006 and into the handle 1004 and provides energy to the tip 1010. The energy tip 1010, here a flat planar blade is coupled to the conductor element. A thin neck region may be used to couple the energy tip 1010 with the conductor element 1012 so that the energy tip 1010 may be bent into a desired shape during use. An optical cladding and/or insulation layer 1014 may be disposed over the conductor element 1012 to isolate it from the optical waveguide 1006. The layer of cladding or insulation 1014 helps to prevent light leakage from the optical waveguide 1006 and also may help prevent energy from leaking from the conductor element 1012.

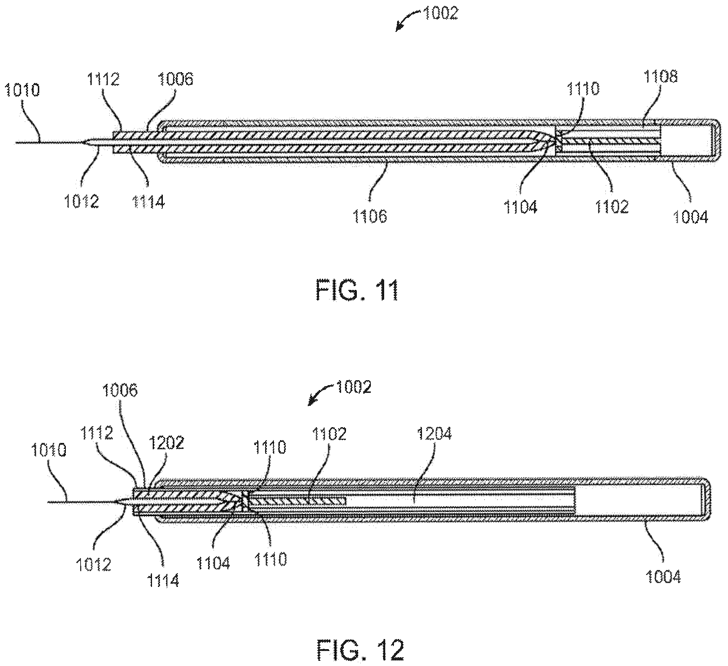

Fig. 11 illustrates a cross-section of the device 1002 in Fig. 10 and highlights the relationship of some of the elements of the device. For example, the energy tip 1010 is coupled with the conductor element 1012 which extends through the waveguide 1006. An outer FEP (fluorinated ethylene propylene) cladding 1112 is disposed over the waveguide 1006 and an inner layer of FEP cladding 1114 is disposed over the conductor element 1012. The waveguide 1106 and conductor element 1012 extend preferably coaxially through the handle 1004. An outer heat sink 1106 maybe coupled to an inside surface of the handle 1004 to help dissipate or transfer heat from the waveguide 1006. This heat sink 1106 may be a metal cylinder extending axially along the longitudinal axis of the handle 1004 or it may be made from other heat conductive materials than can act as a heat sink. A small wire channel 1104 may extend through the proximal end of the waveguide 1006 in order to allow the conductor element 1012 or a wire coupled to the conductor element 1012 to pass through the proximal end of the waveguide 1006 which in this embodiment is preferably a parabolically shaped proximal end similar to those previously described. A light source 1110, in this case a metal core LED printed circuit board (PCB), is coupled to proximal end of the waveguide 1006. The light source 1110 may be any described elsewhere in this specification. An inner heat sink 1108 such as a metal tube may be butt coupled or otherwise coupled to the proximal end of the waveguide 1006 to further help dissipate or transfer heat from the waveguide 1006, and an elongate portion 1102 of the light source 1110 may extend axially away from the LED PCB to the proximal end of the handle 1004 where it may be coupled with a fitting or connector to allow it to be operatively coupled with an external power source, or other service. In this embodiment, the waveguide 1006 has a length that is longer than the length of the inner heat sink 1108. In alternative embodiments, instead of, or in addition to the inner heat sink 1108 butt coupled with a proximal end of the waveguide 1006, a heat sink tube maybe disposed over the waveguide 1006 to partially or fully enclose the waveguide 1006 and dissipate or transfer heat. The assembly may therefore have any combination of a metal tube heat sink, the waveguide 1006, any of the light source embodiments described herein, any of the energy tip embodiments described herein, and any of the handle embodiments described herein.

Fig. 12 illustrates an exemplary embodiment of an illuminated electrosurgical instrument 1002 with an energy tip 1010 that is substantially the same as the embodiment in Fig. 11 with the major difference being that the waveguide 1202 is considerably shorter than the inner heat sink 1204. The inner heat sink 1204 is coupled to the proximal end of the waveguide 1202. In any of the embodiments, the inner heat sink tube 1204, 1108 may also be conductive to provide energy to the light source or the energy tip.

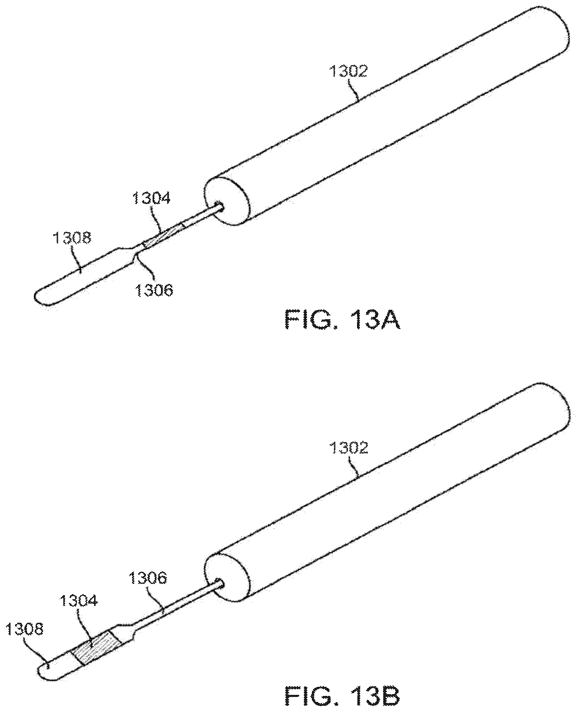

Figs. 13A-13B illustrate exemplary embodiments of an illumination element coupled to an energy tip or conductor element. The illumination element is preferably a waveguide such as those described herein, but may be any illumination element including those disclosed herein. The energy tip similarly may be any energy tip disclosed herein. The energy tip 1308 is coupled to a conductor element 1306 which is coupled to a handle 1302. The waveguide may be a rigid or malleable waveguide 1304 which is coupled to the conductor 1306 in Fig. 13A, while in Fig. 13B the waveguide 1304 may be rigid or malleable and is coupled to the energy tip 1308. This provides lighting that is close to the energy tip 1308. In any embodiment, the energy tip 1308 may be fixedly coupled to the conductor element 1306 or to the handle 1302, or the energy tip 1308 may be releasably coupled to the conductor element 1306 or the handle 1302. The energy tip 1308, conductor element 1306, waveguide 1304, or handle 1302 may be any of the embodiments disclosed herein. The waveguide 1304 may be formed from any of the waveguide materials disclosed herein.

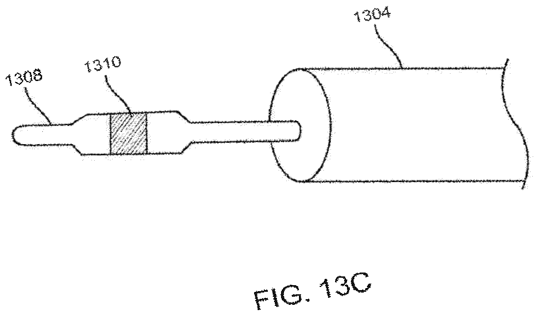

Fig. 13C shows the use of an optional coating on the electrode of Figs. 13A-13B or any of the electrodes described herein. The electrode 1308 is at least partially disposed in the waveguide 1304 which may then be movably coupled to an electrosurgical pencil or other handle. Any of the other electrode and waveguide features described herein may also be used. A portion of the electrode 1308 may be coated with glass and/or may be polished in order to help reflect light emitted from the waveguide 1304. The coating may also provide insulation properties as previously described. The light is preferably reflected toward the tip and toward target work area and this can help minimize glare emitted toward a surgeon or other operator. The coated portion 1310 may be selectively disposed on only a portion of the electrode, or it may be disposed on the entire portion of the electrode, in any desired pattern. The coating may also be on a distal portion adjacent the portions of the electrode where energy is delivered to target tissue, or any portion of the electrode, such as both upper and lower surfaces of the electrode, and/or on the side surfaces of the electrode. The coating may also aid in the distribution of energy densities across the tip by making them more uniform, by minimizing and/or defining portions of the electrode with very high energy densities, or by minimizing and/or defining portions of the electrode with very low energy densities, or any combination thereof.

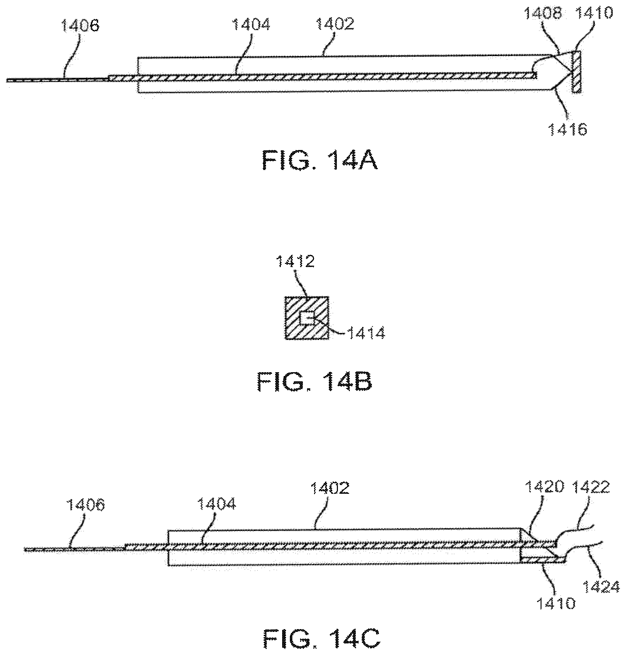

Figs. 14A-14C illustrate alternative embodiments with varying light source positions. Fig. 14A illustrates an energy tip 1406 coupled to a conductor element 1404 which extends through a waveguide 1402. A conductor element such as a wire 1408 is coupled to electrical connection 1412 (see in Fig. 14B) on the light source 1410 and supplies energy to energy tip 1406 such as RF energy. The light source may be any described in this specification. In some embodiments, a single LED 1414 or an array of LEDs may be disposed on the light source 1410. In this embodiment, the light source 1410 is disposed against a proximal portion of the handle and waveguide 1402. A proximal portion 1416 of the waveguide 1402 receives light from the light source 1410. Fig. 14B illustrates an end view of the light source 1410. The light source 1410 is preferably transverse to the longitudinal axis of the waveguide. A single LED may be coaxial with the electrode tip 1406 and the light source 1410 may lie in a plane that is generally orthogonal or otherwise transverse to the axis of the waveguide 1402. The light source may help dissipate or transfer heat into the heat sink that may be surround the waveguide 1402 or that is butt coupled to the light source 1410. Optionally, in any embodiment the waveguide 1402 may be coaxial with the electrode 1406 and/or the conductor element 1404.

Fig. 14C illustrates an alternative embodiment where the light source 1410 is oriented generally parallel to the longitudinal axis of the waveguide 1402 and is disposed adjacent a proximal end of the waveguide 1402. An angled parabolic section 1420 of the waveguide 1402 receives the light from the light source 1410 and transmits it distally toward the energy tip 1406. In this embodiment, a conductor element such as a wire 1422 is coupled to the conductor element 1404 for providing energy to the energy tip 1406. Also, a conductor element 1424 provides power to the light source. Other positions for the LED along the waveguide are contemplated and these embodiments are not intended to be limiting.

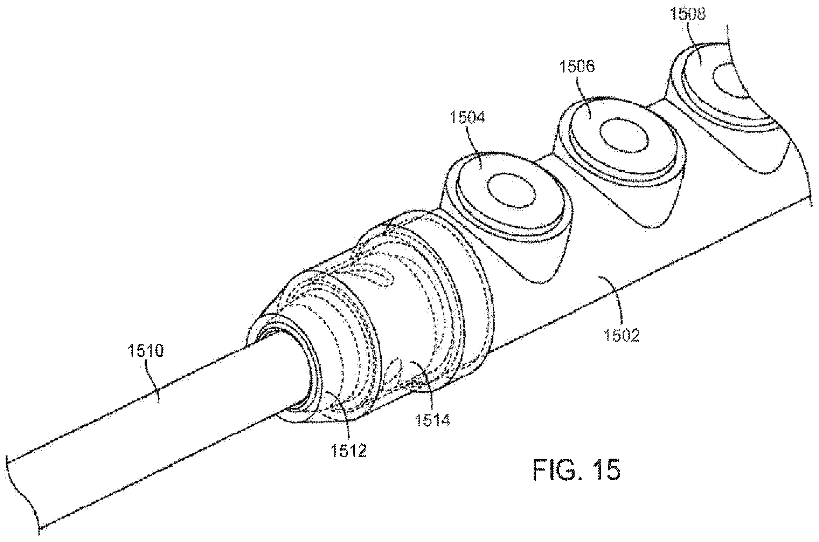

Fig. 15 illustrates an exemplary embodiment of a locking mechanism that may be used with any of the embodiments of movable waveguides or movable energy tips disclosed herein (for example, those illustrated in Figs. 2A-2G). A handle 1502 may include one or more control buttons, here three buttons 1504, 1506, 1508 which may be actuated by the user to turn the energy on or off in various modes. For example one button may be used to turn on and off RF cutting energy to the energy tip. The second button may be used to turn on or turn off coagulating RF energy to the energy tip. The third button may be used to turn on and off illumination from the energy tip without delivering energy to the energy tip. The third button may not be a button, and may instead be a switch such as a pressure sensor or other switch such as a foot switch or slide. Depending on how the illumination element is coupled to the handle, the illumination element (e.g. a LED, an array of LEDs, an optical waveguide, a fiber, etc.) may move relative to the buttons, or it may be fixed. A waveguide 1510 is disposed in the handle 1502 and it may extend outward or inward relative to the handle. The locking mechanism is preferably a "twist-lock" collet style mechanism that clamps circumferentially around an extendable shaft such as a waveguide or energy tip to securely hold it in place at any extended length and rotation. The locking mechanism consists of two pieces, a nose piece 1514 and a collet base piece 1512. When in the unlocked position the shaft or waveguide 1510 can freely rotate, extend, or retract through the inner diameter of the collet. When twisted a predetermined amount, here preferably 90 degrees, in a clockwise motion, the shaft is securely held in place and resists axial movement and rotation. One of skill in the art will appreciate that any amount of rotation and any direction of rotation may be used to lock and unlock the shaft in place.

The collet base piece may have a hollow inner diameter with a split tapered end and designed for a round shaft to be fully inserted through the inner diameter. On the outer diameter of the base piece are two small protrusions (not seen in Fig. 15) that mate with two internal helical grooves on the inner diameter of the nose piece. These protrusions constrain the nose piece from coming free of the base piece and allow the nose piece to rotate a maximum of 90 degrees around the base. As the nose piece is rotated the helical grooves track along on the collet base protrusions and advance the nose piece in a downward direction. The nose piece and base piece have interfering tapers so as the nose piece is tightened against the base piece an inward radial force is created, thus making a secure clamping action around the extendable shaft. This locking mechanism may be used in any of the embodiments describe herein.

In any of the embodiments, the electrode tip may be disposed inside the hollow tube and as described above, the hollow tube may move independently of the electrode tip. Therefore an optical waveguide can slide relative to the length of the electrode tip which gives a surgeon flexibility to position the light at desired positions relative to the electrode tip. This may also allow the surgeon to adjust the spot size of light emitted from the optical waveguide. Moving the optical waveguide distally moves the tip of the waveguide closer to the work surface may therefore decrease the spot size, while retracting the optical waveguide proximally moves the tip of the waveguide away from the work surface and may thereby increase the spot size.

Any of the embodiments of optical waveguide may have a cylindrically shaped optical waveguide. Other shapes for the cross-section of the waveguide may also be employed for any of the embodiments of the optical waveguide such as square, rectangular, elliptical, ovoid, triangular, etc. In one example, flat facets can be used to provide better mixing of light in the waveguide. An odd number of facets is preferred, though an even number of facets may also be used. The number of facets may be determined by the ratio of the input and output sizes discussed earlier. Having more facets will cause the outer waveguide shape to become more like a circle, thus increasing the overall cross section size. Less facets will reduce the overall size of the waveguide. Some embodiments have a tapered optical waveguide such that the proximal portion of the optical waveguide has a larger size than the distal portion. In other embodiments, the optical waveguide may be tapered such that the distal portion of the optical waveguide has a larger size than the proximal portion. In still other embodiments, the central channel of the hollow tube optical waveguide may be used to evacuate smoke, aspirate fluid, and/or delivery treatment materials to and from the surgical field. A vacuum may be applied to a proximal portion of the optical waveguide to draw smoke or other unwanted material out of the surgical field and up into the central channel. A material delivery system may be coupled to a proximal portion of the optical waveguide to deliver material to the surgical field in accordance with many embodiments described throughout this specification.

In other embodiments, the optical waveguide may be a solid rod such that there is no air space or gap between the electrode tip or conductor wire and the inner surface of the optical waveguide. As in previous embodiments, the solid optical waveguide may be fixedly coupled to the handle, or it may be adjustably attached to the handle so that its length may be adjusted to a desired position. The optical waveguide may have a central lumen through which a conducting element, such as a conductor wire or conductor rod is coupled to the electrode, or a proximal portion of the electrode tip may pass through the waveguide to occupy all of the space in the central lumen resulting in a solid waveguide. In some embodiments, this may be accomplished by over molding the waveguide onto the conducting element. The electrode tip may be coupled with the conducting element, or it may be integral with the conducting element. When the electrode tip is integral with the conductor element, the electrode tip is generally not exchangeable with other electrode tips. When the electrode tip is releasably coupled with the conductor element, it may be exchanged with other electrode tips. Preferred embodiments include a non-replaceable electrode tip which can be combined with the adjustable optical waveguide (e.g. slidable or otherwise moving waveguide) feature thereby allowing a user to adjust the light closer to, or away from the work surface for optimal lighting performance. Solid waveguides also provide additional benefits over hollow tube waveguides since they contain more material in the optical waveguide relative to a hollow tube waveguide which allows conduction of a greater amount of light. Additionally, a solid waveguide is structurally stronger than a hollow waveguide. Therefore, a stronger solid waveguide that can carry more light with a smaller profile is possible. The conductor element passing through the solid waveguide also may provide strength to the waveguide.

Fig. 16A shows an exploded view of an exemplary embodiment of an illuminated electrosurgical device having a fully circumferential illumination element. One advantage of this embodiment is that the illumination element and the electrode may be rotated together, thereby ensuring uniform lighting of the target tissue. The illuminated electrosurgical device 1602 includes an anodized aluminum shaft 1600, a cladding 1604, a light source 1606 (referred to here also as an LED board), illumination element 1608 halves (referred to here also as a waveguide), and an electrode blade 1612. The waveguide may be molded as a single unit as described elsewhere in this specification, and therefore may not necessarily have two halves that couple together.

The electrode blade 1612 preferably includes a distal portion which is used to deliver energy (preferably RF energy) to tissue in order to cut or coagulate the tissue. This distal section 1616 may be insulated with a layer of material, here preferably a glass coating. The glass coating is advantageous since it has desirable optical properties and is distal to the waveguide 1608 and therefore helps to ensure that light emitted therefrom is properly reflected from the waveguide toward the surgical target area and minimizes glare back toward the surgeon or other operator. The tip may be insulated by a Teflon (polytetrafluorinated ethylene, PTFE) coating. This coating will scatter and absorb light. The coating on the tip may be a reflective coating. Having a reflective surface on the tip may aid the efficiency of the device by reflecting the light from the waveguide off the surface of the tip towards the target and therefore reduce unnecessary scattering and absorption.

Optionally, in any embodiments disclosed herein, the tip or blade may be made of stainless steel and/or coated. As one example, if the tip is insulated, the tip or blade may be made of stainless steel and then coated. In some embodiments, the coating may start to deteriorate and break off as the blade heats up. In blades or tips where the deterioration of the coating is possible, the tip or blade may be made of ceramic and have a metal frame formed around the tip. A metal wire may also be run around the tip. The metal frame or wire may be made sufficiently thick to prevent degrading.

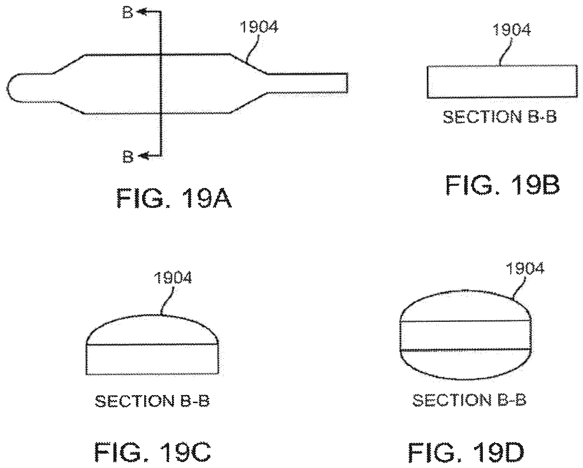

The tip may also have various shapes to aid in dispersion of light. The tip may have a curvature or taper. For example, Fig. 19A illustrates a top view of an electrode 1904. Fig. 19B shows a cross-section of the electrode 1904 taken along the line B-B and shows upper and lower flat planar surfaces while Figs. 19C and 19D show optional convex upper and lower surfaces. Any other electrode cross-section may be used, as well as different tips on the electrode such as a regular flat blade tip, a ball tip, a needle tip, a looped tip or a wire tip. The distal portion may be thin enough to allow an operator to bend the tip in order to conform to the anatomy being treated.

In some embodiments, various tip attachments may be configured to fit over an existing stump to convert the stump to tips having various shapes. In one such embodiment, the stump may be a needle to which a user can attach various sized paddles. Many types of tips may be used during surgical procedures. The tip may be similar to a pin, thin and large. Removable tips or hollow blades may be configured to fit (or stack) over the pin tip making the tip or blade modular. The removable tips or blades may be of any suitable shape and may be configured to frictionally fit over the primary stump or pin tip.

Referring to Fig. 16A, a middle section 1614 of the electrode blade 1612 may also insulated, here preferably with FEP (fluorinated ethylene propylene) in order to prevent energy from leaking out of the electrode along the middle section 1614, and also the FEP provides an index of refraction lower than the index of refraction of the waveguide 1608 thereby helping to prevent or minimize light leakage from the waveguide 1608 due to contact between the waveguide 1608 and electrode blade 1612. A low index of refraction coating or air gaps may also be used in conjunction with or instead of FEP to provide similar effects. A proximal portion of the electrode may include a thin elongate section which serves as a conductor element and allows the electrode to be coupled to wires in the handle (not shown) which may be operably connected to the power supply, preferably an RF generator. The proximal portion of the electrode 1612 may be straight and linear, or it may have an angled or curved section so that a proximal portion of the thin elongate section is off-center, allowing it to pass through the LED board 1606 off center. Optionally, the proximal portion of the electrode 1612 may also be straight and pass through the center of the LED board 1606.

Waveguide 1608 halves maybe snap fit, adhesively bonded, ultrasonically welded together or otherwise joined together, sandwiching the electrode 1612 in between the two waveguide halves. The waveguide 1608 halves form a cylindrical shape around the electrode, thereby illuminating around the electrode 1612. The distal portion of the waveguide 1608 may include a lens, a plurality of lenslets, or other optical features which help shape the light emitted therefrom. In this embodiment, the optical waveguide has an outer surface that is multi-faceted forming a polygon which approximates a cylinder. This extraction surface of the waveguide 1608 may be flat, curved, angled and/or tapered to provide better light directionality, for example with respect to divergence of the light. Having a plurality of facets allows better mixing of light as it passes through the waveguide. Standoffs 1610 in a channel in each half of waveguide 1608 prevent direct contact between the waveguide 1608 and the electrode 1612, thereby minimizing contact and subsequent light loss. The channel in each half of the waveguide 1608 preferably matches the shape of the electrode which lies therein.

The light source, an LED board 1606, includes one or more LEDs for providing light which passes through the waveguide 1608. The LED board 1606 may be any of the LED, light sources, or other light sources described in this specification. The LED may also be parabolically shaped to help focus and deliver the light to the waveguide. In some embodiments, the conductor portion of the electrode may pass through the center of the LED board 1606, or the conductor may pass off center through the LED board 1606.

A layer of cladding 1604 (preferably FEP cladding) may be disposed over the waveguide 1608 and may be heat shrunk down on the two halves, thereby securing the two together. Optionally in conjunction with the FEP cladding 1604 or as an alternative to the FEP cladding 1604, other optical coatings may be used in this or any of the embodiments disclosed herein in order to provide a material with a low index of refraction adjacent the waveguide 1608 to prevent or minimize light loss. Also, an air gap may be disposed against the waveguide to help minimize or prevent light loss since the air gap would provide a lower index of refraction adjacent the waveguide 1608. An outer-most aluminum tube 1600 or other heat conductive material, may then be disposed over the FEP cladding 1064 to keep the components together and/or to serve as a heat sink to remove heat buildup. This tube may also be coupled to the LED board 1606 to dissipate the heat. The entire assembly may then be coupled to a hand-piece and it may telescope in or out of the hand-piece. A locking mechanism (not shown) such as a collet or quarter turn lock may be used to lock the electrode 1612 in position once it has been telescoped into a desired position.

Fig. 16B is an end view of the illuminated electrosurgical device 1602, and Fig. 16C is a cross-section taken along the line B-B in Fig. 16B. Fig. 16C highlights the FEP coated section 1620 and the section of electrode 1622 coupled with standoffs 1610 to minimize direct contact between the electrode 1612 and the waveguide 1608.

In any of the embodiments described herein, the waveguide may also comprise a lens or a lens portion for controlling light delivered from the waveguide. Therefore, the waveguide with or without a lens, and/or a separate lens may be mounted on or otherwise coupled to the LED light source or light source being used. Optionally, and embodiment may therefore include an optical element such as a lens mounted in front of the illumination element such as an LED to direct and shape the light onto the surgical field.

In any of the embodiments described herein, light may be provided to the illumination element (referred to here as the waveguide) by any number of techniques. A light source may be disposed in the handle or adjacent a portion of the waveguide. The light source may be a single LED or multiple LEDs. The LED or multiple LEDs may provide white light, or any desired color. For example, when multiple LEDs are used, the LEDs may provide different colors such as red, green, or blue (RGB) and therefore the multiple LEDs may be adjusted to provide a desired color of light that is input into the waveguide. Thus, the waveguide becomes more important since it may mix different colors of light as the light is transmitted along the length of the waveguide, optionally mixing the different colors of light so that a single uniform color light is delivered to the target. Optionally, multiple colors of light may also be delivered to the target. Multiple colors may be used to provide varying shades of white colored light, or any other desired color which helps the surgeon or operator visualize and distinguish various objects such as tissue in the surgical field. Filters or coatings may be applied to any of the waveguides to filter specific frequencies of energy out. The light of any embodiments described herein may be delivered continuously or strobed.

Alternatively or in combination, the light source may be a fiber optic or fiber bundle in any of the embodiments described herein. For example, a fiber optic may input light to the waveguide from an external source such as a xenon lamp. Light from the external source may be transmitted through the fiber optic or fiber optic bundle through a cable, through the handle, and to the proximal end of the waveguide. The fiber optic or fiber optic bundle may be butted up against the waveguide to provide light to the waveguide and subsequently to a surgical field through the waveguide. A lens or other optical element may be used at the distal end of the fiber optic or fiber bundle to input light to the waveguide with desired optical properties. The light source, for example an external lamp box, may be provided outside the surgical field. Alternatively or in combination, the light source may be a light source in the cable connection. Alternatively or in combination, the light source may be provided in a housing coupled to the cable or to any part of the device.

In any of the embodiments, the waveguide may be made out of a material which has desired optical and mechanical properties. Exemplary materials include acrylic, polycarbonate, cyclo olefin polymer or cylco olefin copolymer. Additionally malleable silicones may be used to form the waveguide so that they may be shaped (plastically deformed) into a desired configuration. Moldable silicone can also be coupled directly to the energy tip to provide a waveguide coupled to the tip and that flexes with the tip when the tip is bent or otherwise flexed. Manufacturers such as Dow Corning and Nusil produce moldable silicones which may be used to form the waveguide.

Additionally, in any of the embodiments described herein, sensors may be integrated into the waveguide or energy tip. These sensors include but are not limited to image sensors such as CMOS or CCD sensors. Sensors could also be thermal or fiber optic to collect spectroscopic information. Sensors may be disposed or otherwise integrated into the handle.

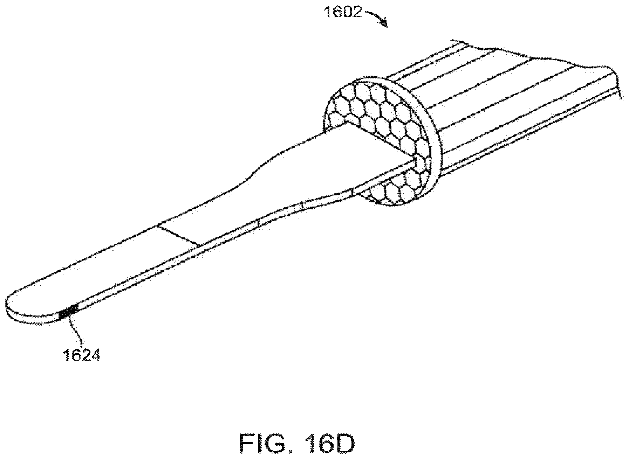

The tip may also include means for sensing to actively measure capacitance, conductance, impedance, inductance and/or any combination of active and/or passive electrical properties (collectively referred to as "the electrical properties") of the tissue in the surgical field. Knowing the electrical properties of the tissue may allow for warning the user if the tip is about to cut through or otherwise damage critical structures. It is also contemplated that alternatively or in combination with the aforementioned sensed properties integrating fiber sensing into the tip to measure temperature spread of the tissue and/or to perform electrical, electrochemical, and/or optical spectroscopic analysis of the tissue. Still other embodiments may include an imaging element such as a camera that can be mounted on the handle or integrated into the sleeve or other portions of the electrosurgical tip. Any of these features may be used or combined with any of the embodiments described herein. Fig. 16D shows an exemplary embodiment of an electrosurgical device 1602 with sensor 1624 integrated therein. The sensor 1624 may for example be an electrical sensor, optical sensor, spectroscopic sensors, and/or thermal sensor. Only one sensor is represented herein, however it will be understood that any number or combination of sensors may be integrated into one or more of the energy tip, waveguide, handle, or combinations thereof.

Still other embodiments may include a handle that has venting features that allow air to circulate through the handle, thereby facilitating cooling of the handle and waveguide.

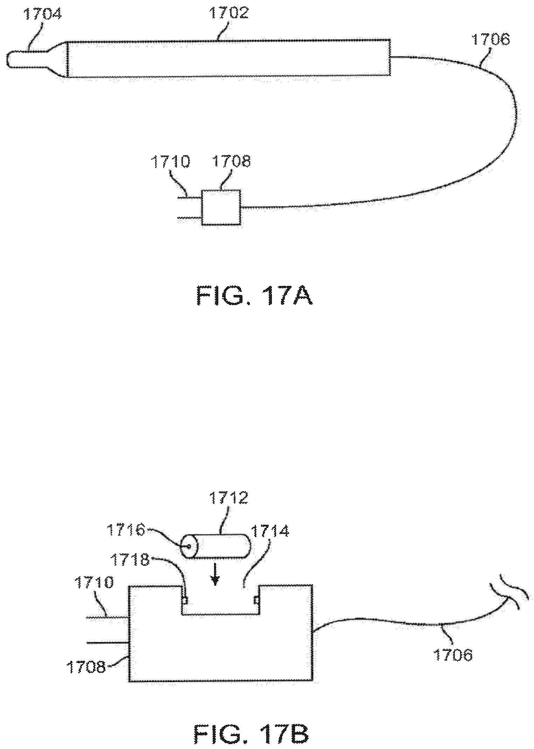

Figs. 17A-17D illustrate use of an optional battery or other power source that provides energy to the illumination element. This optional feature may be used in any of the embodiments described herein.

Fig. 17A illustrates an electrosurgical instrument having a pencil or handle 1702 with an electrode 1704 with or without an illumination element as described in any of the embodiments presented herein. An instrument cable 1706 is fixedly or releasably coupled to the proximal portion of the handle, and the opposite end of the cable 1706 includes a plug or adapter or connector 1708 with electrical connector prongs 1710 for coupling with the electrosurgical generator or any other external box (e.g. controller, light source, power source, etc.).

Fig. 17B highlights features of the plug 1708 which includes a recessed region 1714 that is sized and shaped to receive a battery 1712 or other power source (e.g. capacitor) that can be used to provide power to the lighting/illumination element (e.g. an LED), the electrode, or both. The battery 1712 may be a disposable battery or a rechargeable battery. The battery 1712 may also be a capacitor or other charge storage element. Contacts 1716 on the battery 1712 engage corresponding contacts 1718 in the recessed region 1714 to complete the electrical circuit.

Power to illuminate the illumination element and/or to energize the energy tip 1704 may be delivered from the battery 1712 and/or an external power source connected to the plug 1708 at the connector prongs 1710.

In some embodiments, the battery 1712 disposed within the recessed region 1714 supplies power to the illumination element while the external power source connected to the plug 1708 supplies power to the electrode 1704. The battery 1712 disposed within the recessed region 1714 may also supply power to both the illumination element and the electrode 1708 while the external power source connected to the plug 1708 supplies power to the electrode 1704, the illumination element, both, or neither. Alternatively, the battery 1712 disposed within the recessed region 1714 supplies power to the electrode 1704 while the external power source connected to the plug 1708 supplies power to the illumination element. In some embodiments, with the battery 1712 disposed within the recessed region 1714, the external power sourced connected to the plug 1708 supplies power to the illumination element and to the electrode 1704.

In some embodiments, if the battery 1712 disposed within the recessed region 1714 does not have enough power to supply power to the illumination element, the electrode 1704, both, or neither, the external power source connected to the plug 1708 may supply power to the electrode 1704, the illumination element, both, or neither. Alternatively or in combination with any of the embodiments described within this specification, the battery 1712 may be charged while disposed within the recessed region 1714 of any embodiment and/or within the plug 1708 of any embodiment.

In some embodiments, removing the battery 1712 does not prevent power from getting to the illumination element, the electrode 1704, or both from the external power source. This feature allows a battery to be easily replaced during surgery without interrupting a surgeon who may be using the electrosurgical instrument. In those embodiments with a plug 1708, the portion of the plug 1708 containing the battery 1712 is typically outside of the sterile field thereby further facilitating its easy replacement. The end of the cable 1706 coupled to the plug 1708 may be fixedly or releasably attached to the plug. Thus, the plug may be easily swapped with a new plug having a fresh battery if needed, further facilitating the procedure.

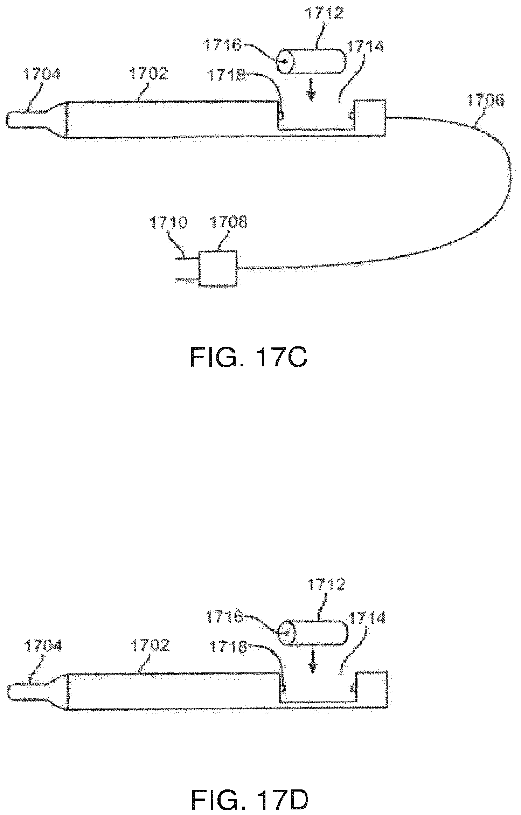

Fig. 17C shows an electrosurgical instrument with a battery 1712 disposed within the handle 1702. The handle 1702 may include a recessed region 1714 that may be sized and shaped to receive a battery 1712 or other power source (e.g. capacitor) that can be used to power the illumination element (e.g. an LED), the electrode, or both. Contacts 1716 on the battery may engage corresponding contacts 1718 in the recessed region 1714 to complete the electrical circuit. The battery 1712 may be a disposable battery or a rechargeable battery. If the battery 1712 is rechargeable, it may optionally be recharged through an instrument cable 1706 which may be fixedly or releasably coupled to the proximal portion of the handle. The cable 1706 may have at the end opposite the end coupled to the proximal portion of the handle, a plug or adapter or connector 1708 with electrical connector prongs 1710 for coupling with the electrosurgical generator or any other external box (e.g. controller, light source, power source, etc.). The cable 1706 may provide power to the electrosurgical instrument with or without the battery 1712 disposed within the handle 1702. This has the advantage of allowing a surgeon to perform surgery even in the absence of the battery 1702, as power supplied through cable may be sufficient to power the illumination element, the electrode, or any other components of the electrosurgical device described herein. In some embodiments, no power will be delivered to the electrosurgical instrument without the battery 1712 disposed within the electrosurgical instrument.

Fig. 17D shows an embodiment similar to that seen in Fig. 17C, but without an instrument cable, making the entire device self-contained, capable of exercising any of the characteristics described herein.

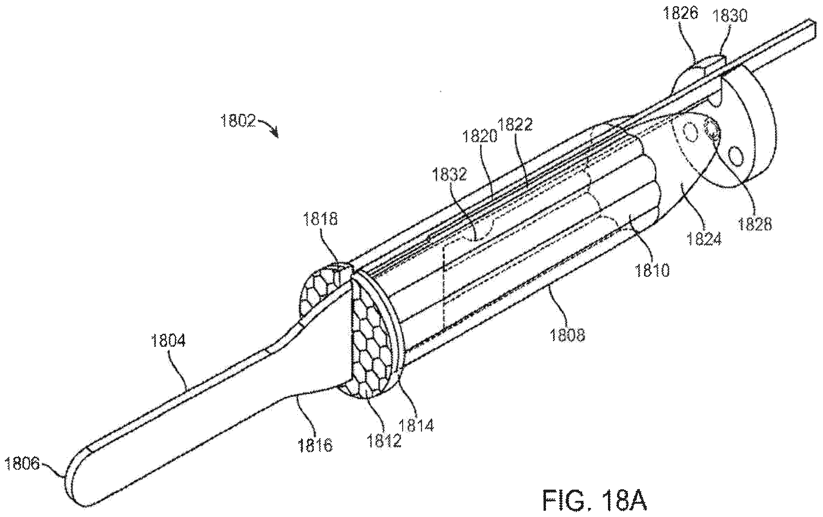

Figs. 18A-18E illustrate still another exemplary embodiment of an illuminated electrosurgical tip 1802. One of skill in the art will appreciate that any of the features described in this embodiment may be used in conjunction with, or substituted for features in any of the other embodiments described herein.

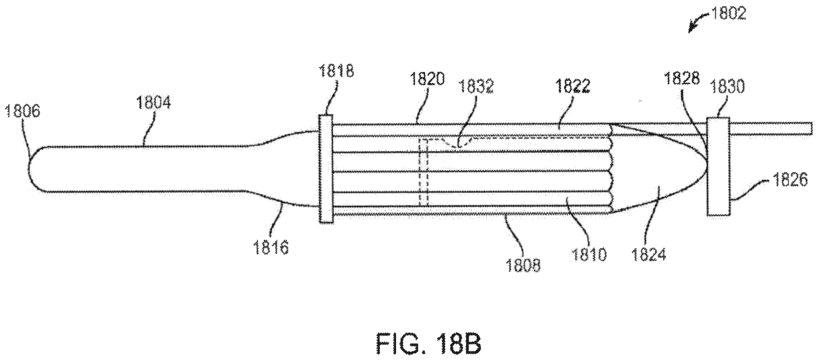

Fig. 18A illustrates an illuminated electrosurgical device 1802 having an electrode 1804 coupled to a waveguide 1808 that extends partially circumferentially around the electrode 1804 and having a light source 1828 on a circuit board 1826 adjacent a proximal end of the waveguide. The electrode 1804 has a distal rounded tip 1806 and may have insulated and/or uninsulated areas similar to those previously described in other embodiments to control delivery of energy to target tissue. The electrode 1804 flares outwardly 1816 (or tapers distally) into a flat planar section which then terminates and only an elongate arm 1822 extends proximally. The elongate arm 1822 may be used as a conductor to deliver energy from an energy source to the electrode tip. The waveguide has a narrow vertically oriented slit 1818 which then transitions into an elongate channel for 1820 for receiving the flat planar section and the elongate arm. The slit 1818 preferably extends along the waveguide, preferably parallel to the longitudinal axis. The slit preferably extends through most of the width of the waveguide, but does not pass all the way therethrough, otherwise the resulting two waveguide halves would fall apart from one another. A short circumferential connector joins both halves of the waveguide. A rounded protrusion 1832 (best seen in Fig. 18B, which is a side view of the exemplary embodiment of Fig. 18A) extends from the elongate arm and is received in a correspondingly shaped recess in the waveguide and prevents axial movement of the electrode relative to the waveguide.

The waveguide 1808 is preferably a non-fiber optic optical waveguide formed as a single integral piece such as by injection molding, though one of skill in the art will appreciate other manufacturing techniques may also be applied (such as milling, chemical etching, etc.). The distal portion of the waveguide may include a plurality of microstructures 1812 for controlling the light extracted therefrom and ensuring that the extracted light has desired optical properties (e.g. divergence, intensity, etc.) in accordance with any of the embodiments described herein. A rim 1814 may be formed around the microstructures 1812 and serves as a surface against which the inner surface of a tube (such as a metal heat sink) may lie or abut. Such tubes have been previously described above and such tubes may each serve as a heat sink. The body of the waveguide 1808 is preferably multi-faceted with a series of outer planar surfaces 1810 forming a polygonal outer surface. This helps with light transmission through the waveguide as the multiple surfaces allow light to bounce off multiple surfaces, thereby providing more mixing of light.

The proximal portion 1824 of the waveguide 1808 is preferably parabolically shaped to help guide light into the waveguide 1808 from the light source 1828 which is preferably an LED or LED array. The parabola is centered over the LED or LED array. The arm 1820 is offset from the central axis of the waveguide 1808 and is received in a slot 1830 in the circuit board 1826.

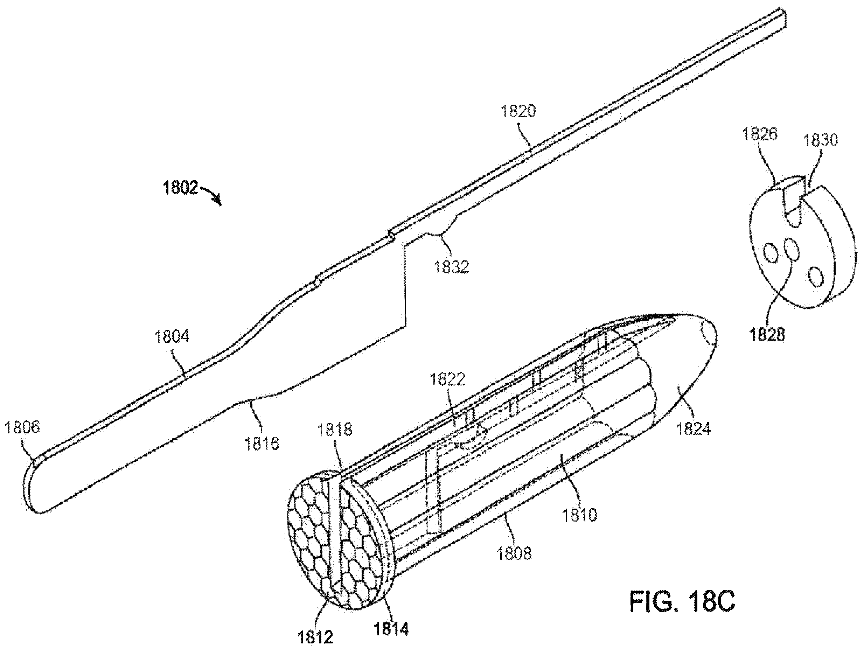

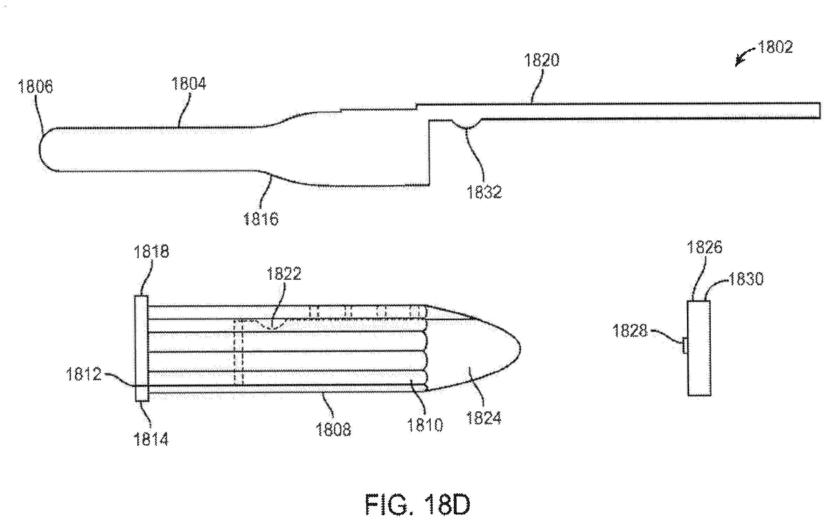

Fig. 18C illustrates an exploded view of the illuminated electrosurgical device 1802, while Fig. 18D shows an exploded side view of the illuminated electrosurgical device1802.

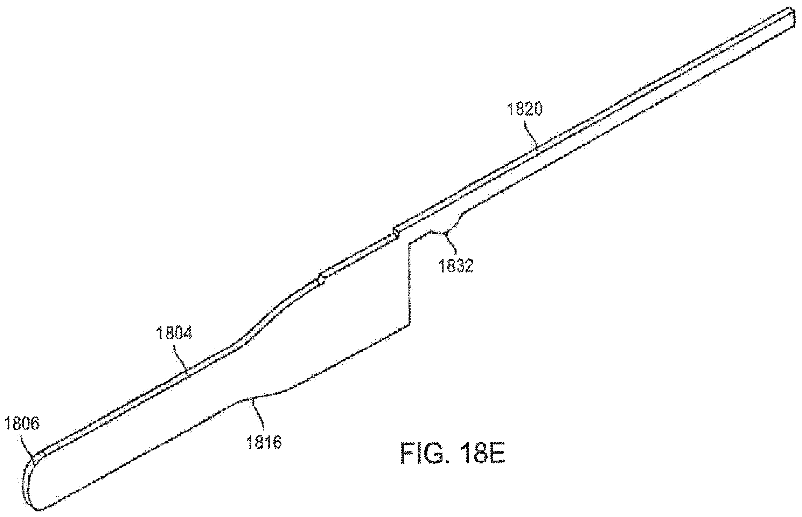

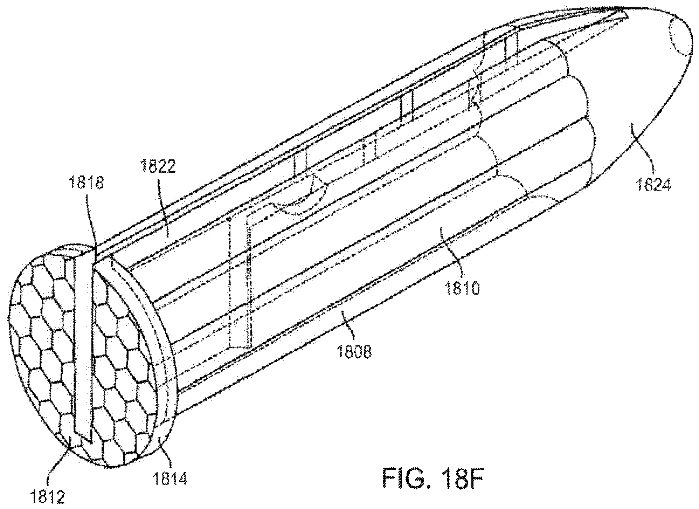

Figs. 18E illustrates a perspective of the electrode 1804 and Fig. 18F shows a perspective view of the waveguide 1808.

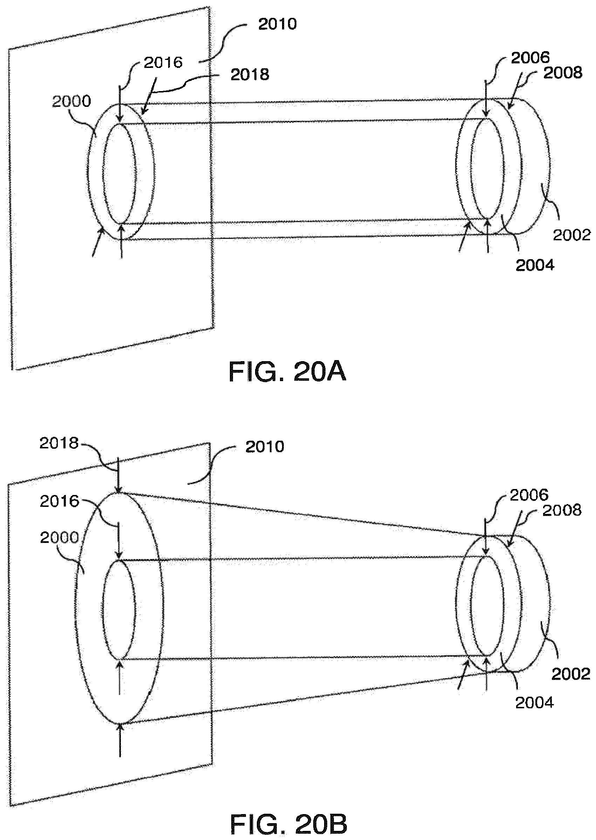

In any of the embodiments herein described, the light cast onto a target region may take on a number of forms. Figs. 20A-20F demonstrate one aspect of how light may be cast onto the target region from the illumination element, namely ways in which the illumination element may cast differently dimensioned light profiles onto the target region (e.g. a surgical site, a portion of a subject's anatomy, etc.). While these illustrated examples emphasize the size of the illuminated profile, other comparable aspects (e.g. the color of the light, the frequency and sequences with which pulses of light may be repeated, the shape of the light profile, etc.) may be appreciated by those of skill in the art.

Figs. 20A-20F illustrate a system for illuminating a target region, the system comprising an illumination element 2002 corresponding to any of the embodiments described throughout this specification, the illumination element further comprising a light emitting surface 2004 with an inner diameter 2006 sized to accommodate a surgical instrument (such as an energy tip, and electrode, etc.) and an outer diameter 2008. In some embodiments the outer diameter 2008 is as small as possible so as to reduce the profile of the illumination element 2002. While the cross-sectional shape of the illumination element 2002 is represented here by a circular shape, any shape may be used including a triangle, a square, a rectangle, a polygon, or any profile which may be represented by a sum of sines and cosines. Likewise, though the term "diameter" is used in the description of Figs. 20A-20F and elsewhere, as used herein it generally refers to a unique measure of a shape. "Diameter" does not refer exclusively to circles or cylinder, though at time it does refer to circles or cylinders. Generally speaking, a diameter is a straight lie passing from one side to another through the center of a shape. As used herein, "diameter" is meant to represent a uniquely identifying dimension or aspect of a shape. Such a uniquely identifying dimension may include but is not limited to a dimension representing the smallest distance between one or more sides or tangents to another one or more sides or tangents within a shape or a dimension representing the largest distance between one or more side or tangents to another one or more sides or tangents within a shape.

The illumination element 2002 emits light through its light emitting surface 2004 and casts a light 2000 onto a target region, the target region residing a distance away from the illumination element 2002 and represented by a plane 2010.

In practice, a surgical instrument (such as an electrode or energy tip) may be disposed within the channel defined by the dimensions of the illumination element 2002 (in this case, the inner diameter 2006) and extend at least partially distally from the illumination element 2002. For the sake of clarity, such a surgical instrument has been left out of Figs. 20A-20F, though the reader should note its presence in some embodiments.

The light 2000 at the plane 2010 of any embodiment described herein may be partially annular or completely annular. The plane 2010 of any embodiment described herein may lie anywhere distal to the illumination element 2002 including but not limited to distal to the illumination element 2002 and proximal to a distal tip of the surgical instrument, distal to the illumination element 2002 and at the distal tip of the surgical instrument, and distal to the illumination element 2002 and distal to the distal tip of the surgical instrument. Therefore, the light 2000 cast by any illumination element 2002 as described herein may be at least partially annular at a plane 2010 in a region proximal to the distal tip of the surgical instrument, the light 2000 cast may be at least partially annular at a plane 2010 at the distal tip of the surgical instrument, and/or the light 200 cast may be at least partially annular at a plane 2010 in a region distal to the distal tip of the surgical instrument.

Fig. 20A shows an exemplary embodiment where the light 2000 at the plane 2010 by the illumination element 2002 has an inner diameter 2016 that is about equal to the inner diameter 2006 of the light emitting surface 2004 of the illumination element 2002 and an outer diameter 2018 that is about equal to the outer diameter 2008 of the light emitting surface 2004 of the illumination element 2002.

Fig. 20B shows an exemplary embodiment where the light 2000 at the plane 2010 by the illumination element 2002 has an inner diameter 2016 that is about equal to the inner diameter 2006 of the light emitting surface 2004 of the illumination element 2002 and an outer diameter 2018 that is greater than the outer diameter 2008 of the light emitting surface 2004 of the illumination element 2002. Though not illustrated, in some embodiments the light 2000 cast at the plane 2010 by the illumination element 2002 may have an inner diameter 2016 that is about equal to the inner diameter 2006 of the light emitting surface 2004 of the illumination element 2002 and an outer diameter 2018 that is less than the outer diameter 2008 of the light emitting surface 2004 of the illumination element 2002.

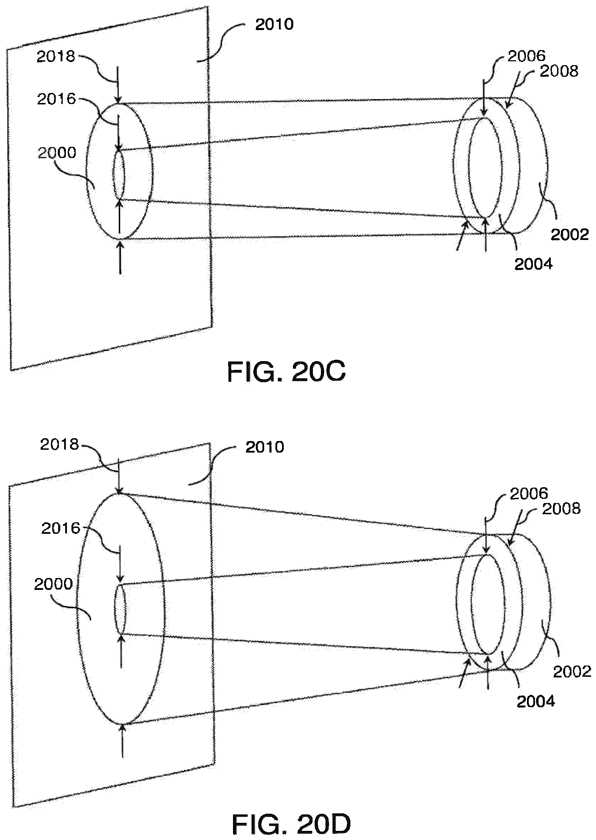

Fig. 20C shows an exemplary embodiment where the light 2000 at the plane 2010 by the illumination element 2002 has an inner diameter 2016 that is less than the inner diameter 2006 of the light emitting surface 2004 of the illumination element 2002 and an outer diameter 2018 that is about equal to the outer diameter 2008 of the light emitting surface 2004 of the illumination element 2002.

For all embodiments described herein, the inner diameter 2016 of the light 2000 at the plane 2010 may be about equal to the diameter of the distal tip of the surgical instrument. The inner profile of the light 2000 at the plane 2010 may at least approximately correspond to an outer profile of the surgical instrument such that no substantial amount of light 2000 is transmitted to the surgical instrument, but to the immediate region near the surgical instrument, such that a minimal amount of light could possibly reflected off of the surgical instrument.

In some embodiments, the inner diameter 2016 of the light 2000 cast at the plane 2010 may be about equal to the dimensions of the distal most end of an energy tip such that the energy tip itself is not illuminated while the region directly surrounding the tip is illuminated. This may be caused by moving the illumination element 2002 closer to or farther from the plane 2010 or energy tip, by moving the energy tip closer to or farther from the plane 2010 or illumination element 2002, and/or by any of methods described herein. In some embodiments, the distance from the illumination element 2002 and the energy tip is fixed permanently and/or temporarily, and in such embodiments the illumination element 2002 may produce a light that illuminates the entire region near the energy tip without substantially illuminating the energy tip itself such that no significant amount of light is absorbed or reflected by the energy tip so as to minimize glare for the user or the subject.

Though not illustrated, in some embodiments the light 2000 cast at the plane 2010 by the illumination element 2002 to have an inner diameter 2016 that is greater than the inner diameter 2006 of the light emitting surface 2004 of the illumination element 2002 and an outer diameter 2018 that is about equal to the outer diameter 2008 of the light emitting surface 2004 of the illumination element 2002.

Fig. 20D shows an exemplary embodiment where the light 2000 cast at the plane 2010 by the illumination element 2002 to have an inner diameter 2016 that is less than the inner diameter 2006 of the light emitting surface 2004 of the illumination element 2002 and an outer diameter 2018 that is greater than the outer diameter 2008 of the light emitting surface 2004 of the illumination element 2002. Though not illustrated, in some embodiments the light 2000 cast at the plane 2010 by the illumination element 2002 to have an inner diameter 2016 that is greater than the inner diameter 2006 of the light emitting surface 2004 of the illumination element 2002 and an outer diameter 2018 that is greater than the outer diameter 2008 of the light emitting surface 2004 of the illumination element 2002. In still other embodiments the light 2000 cast at the plane 2010 by the illumination element 2002 to have an inner diameter 2016 that is smaller than the inner diameter 2006 of the light emitting surface 2004 of the illumination element 2002 and an outer diameter 2018 that is smaller than the outer diameter 2008 of the light emitting surface 2004 of the illumination element 2002.

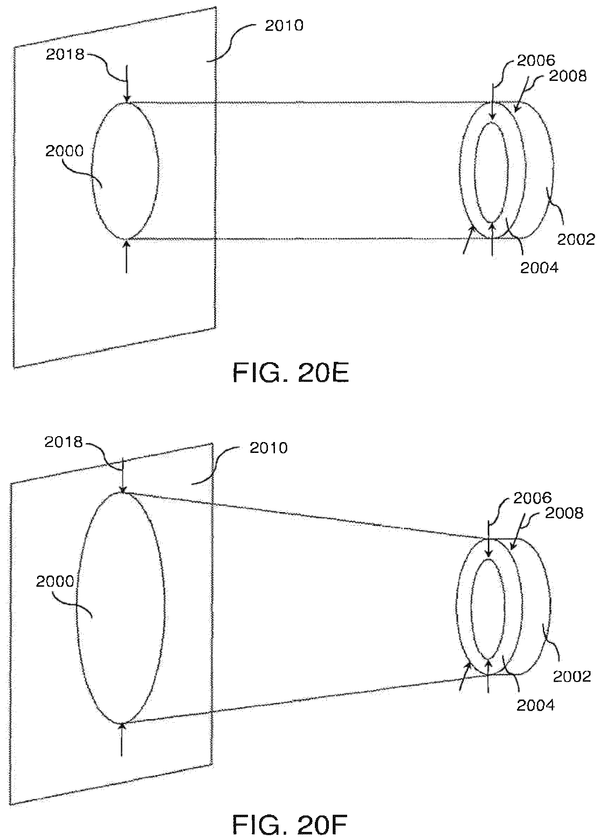

Fig. 20E shows an exemplary embodiment where the light 2000 cast at the plane 2010 illuminates the entire region with an outer profile 1018 about equal to the outer profile 2008 of the light emitting surface 2004 of the illumination element 2002.

Fig. 20F shows an exemplary embodiment where the light 2000 cast at the plane 2010 illuminates the entire region with an outer profile 1018 that is greater than the outer profile 2008 of the light emitting surface 2004 of the illumination element 2002. Though not illustrated, the light 2000 cast at the plane 2010 illuminates the entire region with an outer profile 1018 that is less than the outer profile 2008 of the light emitting surface 2004 of the illumination element 2002. In some embodiments, the energy tip around which the illumination element is disposed may be illuminated. In some embodiments, the energy tip around which the illumination element is disposed is not illuminated. A user may optionally elect, throughout the course of a procedure, to alter one or more characteristics of the light emitted by the illumination element 2002 and they may do so through any of the means described in the body of this specification.

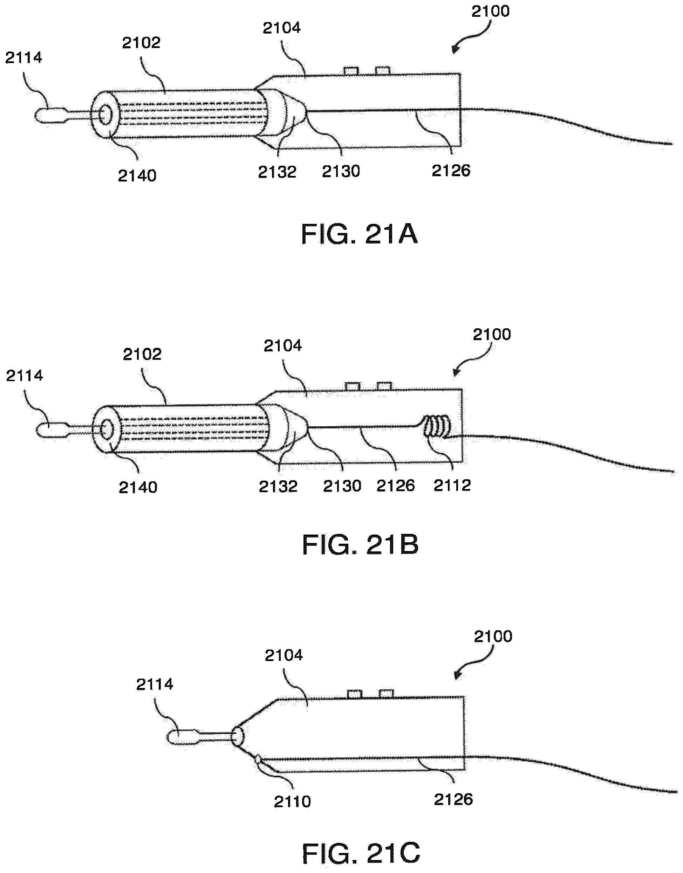

Figs. 21A-21F show an illuminated electrosurgical device 2100 emphasizing an optic fiber 2126 with at least some portion of its length disposed within a hand-piece 2104. Any embodiment may have an optic fiber 2126 disposed coaxial to, parallel to a central axis of, or through any portion of an electrode, an illumination element, a hand-piece.

Fig. 21A shows an illuminated electrosurgical device 2100 comprising a hand-piece 2104 with a proximal and distal end, an illumination element 2102 disposed at the distal end of the hand-piece 2104, an electrosurgical tip 2114 (also referred to herein as an electrode) coupled to the illumination element 2102 near its distal end, and a optic fiber 2126 with at least some portion of its length disposed within the hand-piece 2104 and some portion of its length extending beyond the proximal end of the hand-piece 2104. The illumination element 2102 may be any described herein, having a proximal end 2130 and distal end, and may have near its proximal end 2130 a proximal portion 2132 sized and shaped to disperse light from the optic fiber 2126 such that light emitted from a light extracting surface 2140 at the distal end of the illumination element 2102 is partially or fully circumferential about the electrosurgical tip 2114. Light may be provided from a light source (not shown) at a proximal end of the optic fiber 2126 and transmitted from the proximal end of the optic fiber 2126 to a distal end of the optic fiber 2126, the distal end of the optic fiber 2126 coupled to the illumination element 2102 at its proximal end 2130. The light may be of any kind described herein and may be provided by any light source described herein. The mode or mechanism of coupling the optic fiber 2126 to the illumination element 2102 may be of any sort described herein.

Fig. 21B illustrates an exemplary embodiment of an illuminated electrosurgical device 2100 comprising a hand-piece 2104 with a proximal and distal end, an illumination element 2102 disposed at the distal end of the hand-piece 2104, and a optic fiber 2126 with at least some portion of its length disposed within the hand-piece 2104 and some portion of its length extending beyond the proximal end of the hand-piece 2104, wherein the optic fiber 2126 has a slack-providing portion 2112. For those embodiments in which the illumination element 2102 may travel a length as it telescopes into or out of the hand-piece 2104, the slack-providing portion 2112 provides a length of optic fiber 2126 equal to or greater than the length that the illumination element 2102 may travel.

Fig. 21C shows an exemplary embodiment of an illuminated electrosurgical device 2100 in which a distal end of an optic fiber 2126 couples to an optical element 2110. The optical element 2110 is disposed on a distal end of the hand-piece 2104 near the electrosurgical tip 2114. Light transmitted from a light source disposed at a proximal end of the optic fiber 2126 may be transmitted from the proximal end of the optic fiber 2126 to its distal end where it may then be transmitted to the optical element 2110 which extracts the light and illuminates a target region. The optical element of this or any embodiment may comprise one or more of a lens, a hollow reflector, a gradient lens, a lenslet, a plurality of lenslets, a filter, or a coating for desired optical properties.

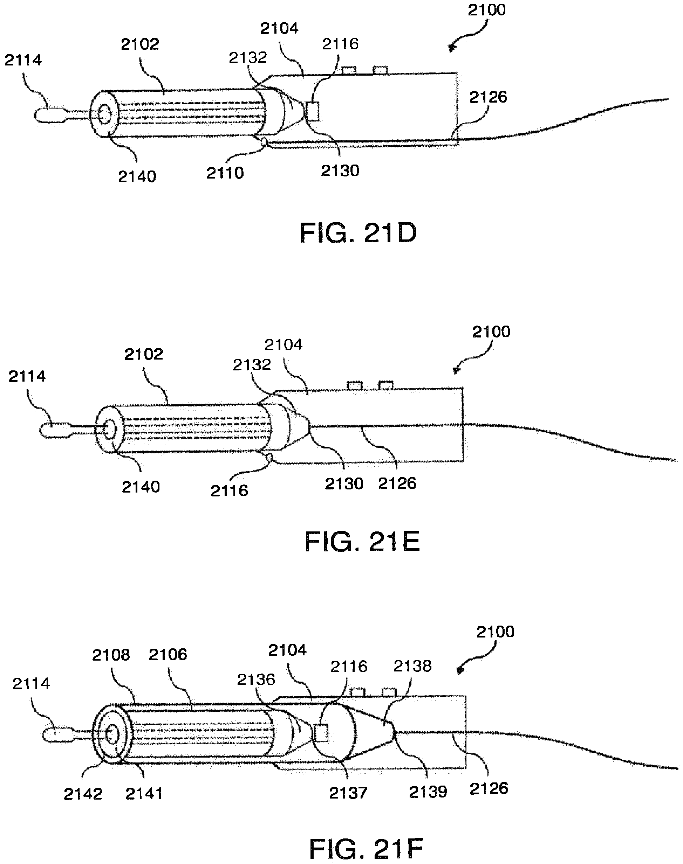

Fig. 21D shows an exemplary embodiment of an illuminated electrosurgical device 2100 comprising two illumination sources: a first source of illumination comprising a light source 2116 housed within a hand-piece 2104 and coupled to a proximal end 2130 of an illumination element 2102 that may be partially or fully circumferential to an electrosurgical tip 2214 and a second source of illumination comprising a light source (not illustrated) coupled to a proximal end of an optic fiber 2126, the optic fiber coupled at its distal end to an optic element 2110 that extracts light from the optic fiber 2126 and illuminates a target region. Thus, one or more light sources may individually provide one or more types of light to the target region. In some embodiments, a partially or fully circumferential illumination element coupled to a light source within the hand-piece may optionally be used in combination with an optical element that is not partially or fully circumferential about the electrosurgical tip, the light source of the optical element disposed outside of the hand-piece and whose light is transmitted to the optical element by a optic fiber.

All embodiments comprising two light sources may have a first light source provide a first light to a target region and have a second light source provide a second light to a target region.

Fig. 21E shows an exemplary embodiment of an illuminated electrosurgical device 2100 comprising two illumination sources. A first illumination source comprises a light source (not illustrated) coupled to a proximal end of an optic fiber 2126, an optic fiber 2126, and an illumination element 2102 whose proximal end 2130 couples to a distal end of the optic fiber 2126. A second illumination source comprises a light source 2116 disposed at a distal end of the hand-piece 2104. The second illumination source may be near the illumination element 2102 or the electrosurgical tip 2114, or both, depending on the embodiment.

Fig. 21F shows an exemplary embodiment of an illuminated electrosurgical device 2100 comprising two illumination sources: a first illumination source comprising a light source 2116 housed within a hand-piece 2104 and coupled to a proximal end 2137 of a first illumination element 2106 that may be partially or fully circumferential to an electrosurgical tip 2214 and a second illumination source comprising a light source (not illustrated) coupled to a proximal end of an optic fiber 2126, the optic fiber coupled at its distal end to a proximal end 2139 of a second illumination element 2108 that may be partially or fully circumferential to the first illumination element 2106. The first illumination element 2106 and the second illumination element 2108 having a first light extracting surface 2141 and a second light extracting surface 2142, respectively, that may be of any type described herein. The first illumination element 2106 and the second illumination element 2108 may comprise a first proximal portion 2136 and a second proximal portion 2138, respectively, shaped to uniformly mix the light received at their respective proximal ends 2137, 2139. The first illumination element 2106 and the second illumination element 2108 may be any illumination element described herein. Though the illustrated embodiment of Fig. 21F shows the second illumination element 2108 partially or fully circumferential about the first illumination element 2106, it should be appreciated that the first illumination element 2106 may be disposed partially or fully circumferential about the second illumination element 2108.

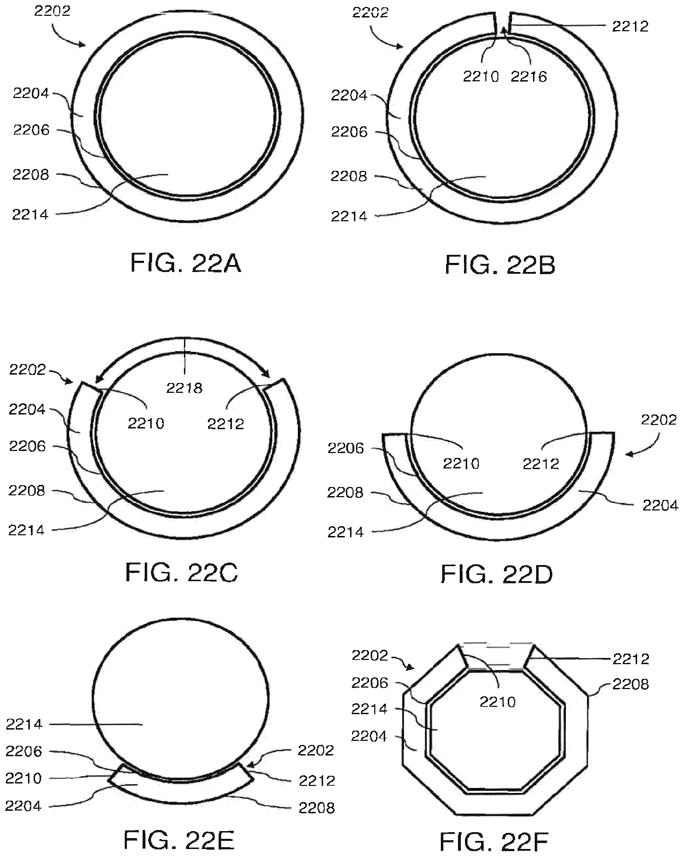

Figs. 22A-22F show exemplary illumination elements that are continuous and at least partially circumferential about a surgical instrument. Though an illuminated element substantially circular shape with a central axis substantially coaxial with a central axis of the surgical instrument has been chosen to illustrate much of the exemplary illumination elements in these figures (for example, Figs. 22A-22E), it should be noted that the illumination element, its inner profile, and its outer profile may individually or collectively take on any cross-sectional shape including a partial or complete circle, a partial or complete oval, a partial or complete ellipse, a partial or complete square, a partial or complete rectangle, a partial or complete polygon, or any partial or complete shape, or any combination thereof and have a central axis that may be coincident with, parallel to, antiparallel to, perpendicular to, intersecting with, or near the central axis of the surgical axis. Here, a central axis may be a uniquely identifying axis such as the an axis passing through the centroid of a shape or an axis passing through the centroid of a shape suggested by the circumference defined by an element (such as the illumination element or the surgical instrument). Likewise, though a substantially circular shape has been chosen to illustrate much of the exemplary surgical instruments in these figures (for example, Figs. 22A-22E), it should be noted that the surgical instrument may take on any cross-sectional shape including a partial or complete circle, a partial or complete oval, a partial or complete ellipse, a partial or complete square, a partial or complete rectangle, a partial or complete polygon, or any partial or complete shape, or any combination thereof.

For any embodiment, the cross-sectional shape, inner profile, and outer profile of the illumination element may individually or collectively be independent of the cross-sectional shape, inner profile, and outer profile of the surgical instrument. The central axis of the illumination element may be offset with respect to the central axis of the surgical instrument. For example, one non-limiting exemplary embodiment of an illumination element with a non-circular profile with a central axis offset from the surgical instrument's central axis is a horizontal ellipse whose central axis is operatively below the central axis of the surgical instrument. In surgical procedures wherein it is critical for users to view the tip, this would confer the advantage of allowing users to see over the device to get a better visualization of the tip by minimizing the top profile of the illumination element. Other such combinations of cross-sectional shapes and axis offsets should be appreciated by one of skill in the art.

The cross-sectional shape of any illumination element or any surgical instrument described herein may change along the length of the feature from its proximal end to its distal end (for instance, the illumination element and/or the surgical instrument may have an overall cone shape) or it may remain substantially constant along the length of the feature from its proximal end to its distal end (for instance, the illumination element and/or the surgical instrument may have an overall cylindrical shape). For all embodiments described herein, any of the continuous illumination elements described herein may be combined with any continuous illumination element described herein.

Fig. 22A shows an exemplary embodiment of a continuous illumination element 2202 disposed at about the perimeter of a surgical instrument 2214. The continuous illumination element 2202 has a light emitting surface 2204, an inner profile 2206, and an outer profile 2208. The light emitting surface 2204 may be of any type described herein. The inner profile 2206 may conform to the profile of the surgical instrument 2214.

Fig. 22B shows an exemplary embodiment of a continuous illumination element 2202 comprising a light emitting surface 204, an inner profile 2206, an outer profile 2208, and a first end 2210 and a second end 2212 defining a gap 2216 therebetween. In this exemplary embodiment, the continuous illumination element 2202 is disposed partially over a surgical instrument 2214. The illumination element 2202 may be flexible enough to allow the illumination element 2202 to be pulled off or placed onto the surgical instrument 2214 at the site of the gap 2216. Conversely, the illumination element 2202 may be flexible enough to allow the surgical instrument 2214 to be placed within or pulled out of the illumination element 2202 at the site of the gap 2216. The illumination element 2202 may be biased toward an operative shape such that over time the illumination element 2202 conforms into the operative shape. The operative shape may be one which corresponds to the profile of the surgical instrument 2214.

Fig. 22C shows an exemplary embodiment of a continuous illumination element 2202 comprising a light emitting surface 2204, an inner profile 2206, an outer profile 2208, a first end 2210, and a second end 2212. In this embodiment, the continuous illumination element 2202 extends over more than half of the perimeter of a surgical instrument 2214. A distance 2218 extends between the first end 2210 and the second end 2212 along the portion of the surgical instrument 2214 not covered by the illumination element 2202. The distance may between the first end 2210 and the second end 2212 may be any value from about 0 millimeters to about the value of the perimeter of the surgical instrument 2214, preferably from about 0 millimeters to about half of the value of the perimeter of the surgical instrument 2214, and more preferably from about 0 millimeters to about one quarter of the value of the perimeter of the surgical instrument 2214.

The illumination element 2202 may be flexible enough to allow the illumination element 2202 to be pulled off or placed onto the surgical instrument 2214 by moving the first end 2210 and the second end 2212 a distance 2218 larger than a diameter of the surgical instrument. Conversely, the illumination element 2202 may be flexible enough to allow the surgical instrument 2214 to be placed within or pulled out of the illumination element 2202 by moving the first end 2210 and the second end 2212 a distance 2218 larger than a diameter of the surgical instrument. The illumination element 2202 may be biased toward an operative shape such that over time the illumination element 2202 conforms into the operative shape. The operative shape may be one which corresponds to the profile of the surgical instrument 2214.

Fig. 22D shows an exemplary embodiment of a continuous illumination element 2202 comprising a light emitting surface 2204, an inner profile 2206, an outer profile 2208, a first end 2210, and a second end 2212. In this embodiment, the continuous illumination element 2202 extends over approximately half of the perimeter of a surgical instrument 2214. In some embodiments, one or more continuous illumination elements 2202 that each extend over approximately half of the perimeter of the surgical instrument 2214 may be used in combination.

Fig. 22E shows an exemplary embodiment of a continuous illumination element 2202 comprising a light emitting surface 2204, an inner profile 2206, an outer profile 2208, a first end 2210, and a second end 2212. The illumination element may conform substantially to the profile of a desired surgical instrument with which it is meant to be used in conjunction. In this embodiment, the continuous illumination element 2202 extends over less than half of the perimeter of a surgical instrument 2214. In some embodiments, one or more continuous illumination elements 2202 that each extends less than half of the perimeter of the surgical instrument 2214 may be used in combination.

Fig. 22F shows an exemplary embodiment of a continuous illumination element 2202 having a polygonal profile disposed over a surgical device 2214. Though the polygonal profile of the illustrated embodiment is octagonal, any shape may be used. The polygonal continuous illumination element 2202 comprises a light emitting surface 2204, an inner profile 2206, and outer profile 2208, a first end 2210, and a second end 2212. The inner profile 2206 and the outer profile 2208 may have different shapes for profiles, may have the same profile shape but offset linearly and/or rotated, or may be substantially similar in shape.

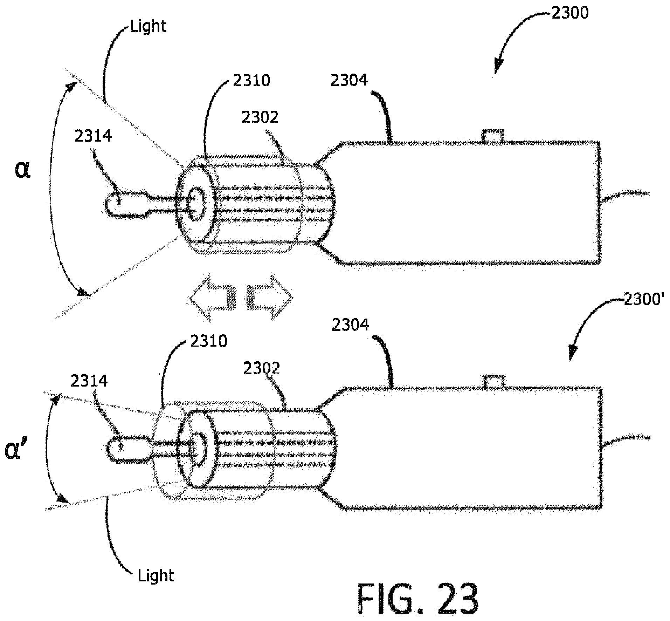

Optionally, in any embodiments described herein a movable shroud may be provided. In an example embodiment, a movable shroud may be positioned around the waveguide to permit adjustment of the angle of divergence of the light emitted by the waveguide. Fig. 23 illustrates an example embodiment in which an electrosurgical instrument 2300 includes a handle 2304, a waveguide 2302 and a tip 2314 extending from the handle 2304, through the waveguide 2302 and distally outward for operation. A movable shroud 2310 is positioned to surround the waveguide 2302. Double arrows below the device 2300 indicate the movability of the movable shroud 2310 so as to adjust an angle of divergence a. The movable shroud 2310 is in its most retracted position on device 2300 providing for a maximum angle of divergence a. In an example implementation, the movable shroud 2310 may be provided with a reflective surface inside. For example, the inside of the shroud can also be polished/coated to reduce absorption and increase the output of the device. The device as shown at 2300' is shown with the movable shroud 2310 extended distally to reduce the angle of divergence o'.

In some procedures in which an electrosurgical instrument is used, it may be desired to view an area of operation at different angles, such as in a direction behind the direction of illumination of most of the light from the waveguide. For example, adenoid procedures would advantageously use a device that illuminates areas behind the electrosurgical tip. Optionally, in any embodiments described herein, a mirror attachment may be provided. In an example embodiment, an electrosurgical device 2400 may be provided with a mirror attachment 2422 capable of slipping over a blade 2414 to provide an illuminated mirror. The mirror attachment 2422 may be mounted at a distal end of a hollow post 2420 configured to slide over the ES tip 2414. The hollow post 2420 slides frictionally over the blade 2414 and the light from the electrosurgical instrument 2400 is directed to the reflective surface of the mirror 2422 to reflect in a substantially opposite direction as shown at 2400'. The hollow illuminated mirror post 2420 may also insulate the blade 2414 providing safety to the patient.

It is noted that any embodiments described above may include lenslets or microstructures. The lenslets or microstructures may be disposed on the distal end of various embodiments of illumination elements. The lenslets or microstructures may be alternative light extraction surfaces providing various ways to control the light emitted from the illumination element, such as by providing options for diffusing or focusing the emitted light. In example embodiments of lenslets or microstructures, including those, for example, described above with reference to Figs. 3A, 4C, 9, 10, 16A, 18B, and 21C, the lenslets or microstructures may be configured to be removable and interchangeable to configure the illumination element to emit light in different ways according to specific applications. The waveguide light extraction surface may be provided as non-structured or flat so as to receive a desired removable lenslet or microstructure module.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.
Also the following examples pertain to the scope of the present disclosure:
1. An illuminated electrosurgical instrument, said instrument comprising:
   a handle with a proximal portion and a distal portion;
   an illumination element coupled to the handle near the distal portion thereof; and
   an electrosurgical tip coupled to the illumination element, and
   wherein the illumination element extends continuously and at least partially circumferentially about the electrosurgical tip.
2. The device of example 1, wherein the illumination element casts a beam of light that is continuous and at least partially annular.
3. The device of example 2, wherein the beam of light is continuous and at least partially annular distal to the illumination element and proximal to a distal tip of the electrosurgical tip.
4. The device of example 2, wherein the beam of light is continuous and at least partially annular distal to the illumination element and at the distal tip of the electrosurgical tip.
5. The device of example 2, wherein the beam of light is continuous and at least partially annular distal to the illumination element and distal to the distal tip of the electrosurgical tip.
6. The device of example 1, wherein the illumination element comprises an optical waveguide.
7. The device of example 1, wherein the illumination element comprises an organic light emitting diode (OLED).
8. The device of example 1, wherein the illumination element comprises one or more discrete light emitting diodes (LEDs).
9. The device of example 1, wherein the illumination element comprises a plurality of optic fibers.
10. The device of example 1, wherein a cross-sectional shape of the illumination element is selected from one of the following: a partial or complete circle, a partial or complete oval, a partial or complete ellipse, a partial or complete square, a partial or complete rectangle, and a partial or complete polygon.
11. The device of example 1, wherein the illumination element is adjustably coupled to the handle, and wherein actuation of the illumination element in a first direction moves the illumination element toward the proximal portion of the handle, and wherein actuation of the illumination element in a second direction opposite the first direction moves the illumination element toward the distal portion of the handle.
12. The device of example 11, wherein the electrosurgical tip is adjustably coupled to the illumination element, and wherein actuation of the electrosurgical tip in a first direction moves the electrosurgical tip toward the proximal portion of the handle, and wherein actuation of the electrosurgical tip in a second direction opposite the first direction moves the electrosurgical tip toward the distal portion of the handle.
13. The device of example 1, wherein the electrosurgical tip is adjustably coupled to the illumination element, and wherein actuation of the electrosurgical tip in a first direction moves the electrosurgical tip toward the proximal portion of the handle, and wherein actuation of the electrosurgical tip in a second direction opposite the first direction moves the electrosurgical tip toward the distal portion of the handle.
14. The device of example 1, wherein the electrosurgical tip is removably coupled to the illumination element.
15. The device of example 1, further comprising a light source, the light source coupled to a proximal end of the illumination element
16. The device of example 15, further comprising a battery disposed within the handle, the battery supplying power to the light source.
17. The device of example 1, comprising a first mechanical switch configured to apply power to the electrosurgical tip.
18. The device of example 1, comprising a motion sensor configured to detect motion of the electrosurgical device and to switch on electrical power to the light source.
19. The device of example 18, wherein the motion sensor is an accelerometer.
20. The device of example 1, comprising a movable shroud circumferentially surrounding the illumination element and configured to move axially in a distal or proximal direction to adjust an angle of divergence of light extending from the distal end of the illumination element.
21. The device of example 1, where the electrosurgical tip includes a reflective coating made of an insulating material.
22. The device of example 1, where the electrosurgical tip is made of a ceramic material and a metallic frame around an edge of the electrosurgical tip for conducting current during operation.
23. The device of example 1, further comprising a mirror attachment having a hollow post and an attached mirror where the hollow post is configured to slip over the electrosurgical tip to mount the attached mirror to the device and to operate as an illuminated mirror.
24. The device of example 1, where the electrosurgical tip is a removable electrosurgical tip configured to mount on a stump extending distally from the device, where the stump is configured to receive one of a plurality of electrosurgical tips having different configurations for different functions.
25. The device of example 24, wherein the stump is a needle.
26. A method for illuminating a surgical target, said method comprising:
   providing an electrosurgical tip having a circumferential illumination element;
   illuminating the surgical target with light from the illumination element; and
   moving the illumination element toward or away from the surgical target, thereby adjusting the illumination on the surgical target.
27. The method of example 26, further comprising replacing the electrosurgical tip with a different electrosurgical tip.
28. The method of example 26, further comprising locking one or more of the electrosurgical tip or the illumination element after illumination adjustment.
29. An illuminated electrosurgical instrument, said instrument comprising:
   a handle;
   an illumination element coupled to the handle;
   an electrosurgical tip coupled to the illumination element; and
   an optical element disposed at least partially and circumferentially around the electrosurgical tip and is coupled to the illumination element,
   wherein the optical example delivers a continuous, annular beam of light to a target.
30. The device of example 29, wherein the illumination element comprises an organic light emitting diode (OLED).
31. The device of example 29, wherein the illumination element comprises one or more discrete light emitting diodes (LEDs).
32. The device of example 29, wherein the illumination element comprises a plurality of optic fibers.
33. The device of example 29, wherein the illumination element is adjustably coupled to the handle, and wherein actuation of the illumination element in a first direction moves the illumination element toward the proximal portion of the handle, and wherein actuation of the illumination element in a second direction opposite the first direction moves the illumination element toward the distal portion of the handle.
34. The device of example 33, wherein the electrosurgical tip is adjustably coupled to the illumination element, and wherein actuation of the electrosurgical tip in a first direction moves the electrosurgical tip toward the proximal portion of the handle, and wherein actuation of the electrosurgical tip in a second direction opposite the first direction moves the electrosurgical tip toward the distal portion of the handle.
35. The device of example 29, wherein the electrosurgical tip is adjustably coupled to the illumination element, and wherein actuation of the electrosurgical tip in a first direction moves the electrosurgical tip toward the proximal portion of the handle, and wherein actuation of the electrosurgical tip in a second direction opposite the first direction moves the electrosurgical tip toward the distal portion of the handle.
36. The device of example 29, wherein the electrosurgical tip is removably coupled to the illumination element.
37. The device of example 29, wherein the optical element comprises one or more of a lens, a hollow reflector, a gradient lens, a lenslet, a plurality of lenslets, a filter, or a coating for desired optical properties.
38. The device of example 29, wherein the optical element is configured to receive one of a plurality of removable lenslets configured to provide different focusing and diffusion features.
39. The device of example 29, further comprising:
   at least one lumen formed to extend axially within the illumination element from a distal end of the illumination element proximally to a connection to a vacuum pump configured to aspirate air from the area of operation; and
   a liquid filter configured to filter liquid from the aspirated air entering the lumen.
40. The device of example 29, further comprising a light source, the light source coupled to a proximal end of the illumination element
41. The device of example 29, further comprising a battery disposed within the handle, the battery supplying power to the light source.
42. The device of example 29, wherein the optical element is concentric to the electrosurgical tip.
43. A method for illuminating a surgical target, said method comprising:
   providing an electrosurgical device having an optical element disposed at least partially and circumferentially around an electrosurgical tip; and
   illuminating the surgical target with light from the optical element.
44. The method of example 43, further comprising moving the optic fiber toward or away from the surgical target, thereby adjusting the illumination on the surgical target.
45. An illuminated electrosurgical instrument, said instrument comprising:
   a handle with a proximal portion and a distal portion;
   an electrosurgical tip coupled to the handle near the distal portion thereof; and
   an optic fiber with a proximal portion and a distal portion, the distal portion of the optic fiber coupled to the proximal portion of the handle, wherein light is delivered by the optic fiber to a target.
46. The device of example 45, wherein the proximal portion of the optic fiber extends proximally outside the proximal portion of the handle.
47. The device of example 46, wherein the proximal portion of the optic fiber is coupled to a light source.
48. The device of example 47, wherein the light source comprises an LED, a plurality of LEDs, a laser, a xenon lamp, or any combination thereof.
49. The device of example 45, wherein the optic fiber may comprise a plurality of optic fibers.
50. The device of example 45, wherein the illumination element is adjustably coupled to the handle, and wherein actuation of the illumination element in a first direction moves the illumination element toward the proximal portion of the handle, and wherein actuation of the illumination element in a second direction opposite the first direction moves the illumination element toward the distal portion of the handle.
51. The device of example 50, wherein the electrosurgical tip is adjustably coupled to the illumination element, and wherein actuation of the electrosurgical tip in a first direction moves the electrosurgical tip toward the proximal portion of the handle, and wherein actuation of the electrosurgical tip in a second direction opposite the first direction moves the electrosurgical tip toward the distal portion of the handle.
52. The device of example 45, wherein the electrosurgical tip is adjustably coupled to the illumination element, and wherein actuation of the electrosurgical tip in a first direction moves the electrosurgical tip toward the proximal portion of the handle, and wherein actuation of the electrosurgical tip in a second direction opposite the first direction moves the electrosurgical tip toward the distal portion of the handle
53. A method for illuminating a surgical target, said method comprising:
   providing an electrosurgical device having a handle and an optic fiber, at least a portion of the optic fiber disposed within the handle, the fiber optic coupled to a light source; and
   illuminating the surgical target with light from the optic fiber.
54. The method of example 53, further comprising moving the optic fiber toward or away from the surgical target, thereby adjusting the illumination on the surgical target.
55. An illuminated electrosurgical instrument, said instrument comprising:
   a handle with a proximal end and a distal end;
   an electrosurgical tip coupled to the handle near the distal end thereof;
   a first illumination element with a proximal end and a distal end, the first illumination element disposed continuously and circumferentially around the electrosurgical tip; and
   a second illumination element with a proximal end and a distal end, the second illumination element disposed on the handle near the distal end thereof,
   wherein the first illumination element delivers a first light to a target and the second illumination element delivers a second light to the target, and
   wherein the first light is a continuous, annular beam of light extending around the electrosurgical tip.
56. The device of example 55, wherein the first illumination element comprises an optical waveguide, one or more LEDs, an OLED, a plurality of optic fibers, or any combination thereof.
57. The device of example 55, wherein the second illumination element comprises an optical waveguide, one or more LEDs, an OLED, one or more optic fibers, or any combination thereof.
58. The device of example 55, wherein a first light source disposed within the handle provides the first light for the first illumination element.
59. The device of example 55, wherein an external light source provides the first light for the first illumination element.
60. The device of example 59, wherein the external light source is an LED, a plurality of LEDs, a laser, a xenon lamp, or any combination thereof.
61. The device of example 55, wherein a second light source disposed within the handle provides the second light for the second illumination element.
62. The device of example 55, wherein an external light source provides the second light for the second illumination element.
63. The device of example 62, wherein the external light source is an LED, a plurality of LEDs, a laser, a xenon lamp, or any combination thereof.
64. The device of example 55, wherein the first illumination element and the second illumination element are concentric.
65. The device of example 55, wherein the first illumination element is adjustably coupled to the handle, and wherein actuation of the illumination element in a first direction moves the illumination element toward the proximal portion of the handle, and wherein actuation of the illumination element in a second direction opposite the first direction moves the illumination element toward the distal portion of the handle.
66. The device of example 65, wherein the electrosurgical tip is adjustably coupled to the first illumination element, and wherein actuation of the electrosurgical tip in a first direction moves the electrosurgical tip toward the proximal portion of the handle, and wherein actuation of the electrosurgical tip in a second direction opposite the first direction moves the electrosurgical tip toward the distal portion of the handle.
67. The device of example 55, wherein the electrosurgical tip is adjustably coupled to the first illumination element, and wherein actuation of the electrosurgical tip in a first direction moves the electrosurgical tip toward the proximal portion of the handle, and wherein actuation of the electrosurgical tip in a second direction opposite the first direction moves the electrosurgical tip toward the distal portion of the handle.
68. A method for illuminating a surgical target, said method comprising:
   providing an electrosurgical device having a first illumination element and a second illumination element;
   illuminating the surgical target with light from the first illumination element; and
   illuminating the surgical target with light from the second illumination element.
69. The method of example 68, further comprising moving the first illumination element toward or away from the surgical target, thereby adjusting the illumination on the surgical target.
70. An illuminated electrosurgical instrument, said instrument comprising:
   a handle with a proximal portion and a distal portion;
   an illumination element coupled to the handle near the distal portion thereof; and
   an electrosurgical tip coupled to the illumination element,
   wherein the illumination element extends continuously and circumferentially about the electrosurgical tip, and
   wherein the illumination element comprises a slot disposed at least partially through a thickness of the illumination element, the slot extending axially and at least partially along a length of the illumination element, and
   wherein at least a portion of the electrosurgical tip is disposed in the slot.

## Claims

1. An illuminated electrosurgical instrument (1002), said instrument (1002) comprising:
a handle (12; 204; 1004; 1302; 1502; 2304) with a proximal portion and a distal portion;
an electrosurgical tip (1802; 2114; 2214) extending distally of the handle (12; 204; 1004; 1302; 1502; 2304), wherein the electrosurgical tip (1802; 2114; 2214) is configured to perform electrosurgery using a radiofrequency (RF) energy, and
an optical waveguide (202; 702; 1006; 1202; 1304; 1402; 15101; 1808; 2302) that extends continuously and at least partially circumferentially about the electrosurgical tip (1802; 2114; 2214), wherein the optical waveguide (202; 702; 1006; 1202; 1304; 1402; 15101; 1808; 2302) has a proximal end and a distal end;
a plurality of light sources (216; 306, 324; 416, 422; 606, 652; 706; 1110; 1410; 1606; 1828; 2116) optically coupled to the proximal end of the optical waveguide (202; 702; 1006; 1202; 1304; 1402; 15101; 1808; 2302), wherein the plurality of light sources (216; 306, 324; 416, 422; 606, 652; 706; 1110; 1410; 1606; 1828; 2116) is configured to generate light at a plurality of colors; and
an illumination switch (206) that is configured to control the plurality of light sources (216; 306, 324; 416, 422; 606, 652; 706; 1110; 1410; 1606; 1828; 2116), wherein the illumination switch (206) comprises a slidable switch that is configured to control at least one of an intensity of the light or a color of the light based on how far the slidable switch has been slid, and
wherein the optical waveguide (202; 702; 1006; 1202; 1304; 1402; 15101; 1808; 2302) is configured to transmit the light from the plurality of light sources (216; 306, 324; 416, 422; 606, 652; 706; 1110; 1410; 1606; 1828; 2116).

2. The illuminated electrosurgical instrument (1002) of claim 1, wherein the optical waveguide (202; 702; 1006; 1202; 1304; 1402; 15101; 1808; 2302) is configured to deliver the light with a plurality of discrete colors.

3. The illuminated electrosurgical instrument (1002) of claim 1 or 2, wherein the optical waveguide (202; 702; 1006; 1202; 1304; 1402; 15101; 1808; 2302) is configured to mix the light generated by the plurality of light sources (216; 306, 324; 416, 422; 606, 652; 706; 1110; 1410; 1606; 1828; 2116) to form a beam of light with a single uniform color that is a mixture of the plurality of colors of respective lights generated by the plurality of light sources (216; 306, 324; 416, 422; 606, 652; 706; 1110; 1410; 1606; 1828; 2116).

4. The illuminated electrosurgical instrument (1002) of claim 3, wherein the illumination switch (206) is configured to control the color of the light.

5. The illuminated electrosurgical instrument (1002) of claim 4, wherein the illumination switch (206) is configured to slide relative to the handle (12; 204; 1004; 1302; 1502; 2304) to control the color of the light.

6. The illuminated electrosurgical instrument (1002) of any one of claims 1-5, wherein the plurality of colors is used to provide varying shades of white light.

7. The illuminated electrosurgical instrument (1002) of any one of claims 1-6, wherein the optical waveguide (202; 702; 1006; 1202; 1304; 1402; 15101; 1808; 2302) comprises:
a filter that is configured to filter one or more frequencies of light out of a beam of light; or
a coating that is configured to filter one or more frequencies of light out of the beam of light.

8. The illuminated electrosurgical instrument (1002) of any one of claims 1-7, wherein the plurality of light sources (216; 306, 324; 416, 422; 606, 652; 706; 1110; 1410; 1606; 1828; 2116) is configured to generate the light in a strobed manner.

9. The illuminated electrosurgical instrument () of any one of claims 1-8, wherein the plurality of light sources (216; 306, 324; 416, 422; 606, 652; 706; 1110; 1410; 1606; 1828; 2116) is located in the handle (12; 204; 1004; 1302; 1502; 2304).

10. The illuminated electrosurgical instrument (1002) of any one of claims 1-8, wherein the plurality of light sources (216; 306, 324; 416, 422; 606, 652; 706; 1110; 1410; 1606; 1828; 2116) is located external to the handle (12; 204; 1004; 1302; 1502; 2304).

11. The illuminated electrosurgical instrument (1002) of any one of claims 1-10, wherein the plurality of light sources (216; 306, 324; 416, 422; 606, 652; 706; 1110; 1410; 1606; 1828; 2116) comprises a first light emitting diode (LED), a second LED, and a third LED, and
wherein the first LED is configured to generate red light, the second LED is configured to generate green light, and the third LED is configured to generate blue light.

12. The illuminated electrosurgical instrument (1002) of any one of claims 1-11, wherein the optical waveguide (202; 702; 1006; 1202; 1304; 1402; 15101; 1808; 2302) is adjustably coupled to the handle (12; 204; 1004; 1302; 1502; 2304), and wherein actuation of the optical waveguide (202; 702; 1006; 1202; 1304; 1402; 15101; 1808; 2302) in a first direction moves the optical waveguide (202; 702; 1006; 1202; 1304; 1402; 15101; 1808; 2302) toward a proximal portion of the handle (12; 204; 1004; 1302; 1502; 2304), and wherein actuation of the optical waveguide (202; 702; 1006; 1202; 1304; 1402; 15101; 1808; 2302) in a second direction opposite the first direction moves the optical waveguide (202; 702; 1006; 1202; 1304; 1402; 15101; 1808; 2302) toward a distal portion of the handle (12; 204; 1004; 1302; 1502; 2304).

13. The illuminated electrosurgical instrument (1002) of one of claim 12, wherein the electrosurgical tip (1802; 2114; 2214) is adjustably coupled to the optical waveguide (202; 702; 1006; 1202; 1304; 1402; 15101; 1808; 2302), and wherein actuation of the electrosurgical tip (1802; 2114; 2214) in a first direction moves the electrosurgical tip (1802; 2114; 2214) toward the proximal portion of the handle (12; 204; 1004; 1302; 1502; 2304), and wherein actuation of the electrosurgical tip (1802; 2114; 2214) in a second direction opposite the first direction moves the electrosurgical tip (1802; 2114; 2214) toward the distal portion of the handle (12; 204; 1004; 1302; 1502; 2304).

14. The illuminated electrosurgical instrument (1002) of any one of claims 1-13, comprising a motion sensor configured to detect motion of the illuminated electrosurgical instrument (1002) and, responsive to detection of motion, to switch on electrical power to the plurality of light sources (216; 306, 324; 416, 422; 606, 652; 706; 1110; 1410; 1606; 1828; 2116).

15. The illuminated electrosurgical instrument (1002) of claim 14, wherein the motion sensor is an accelerometer.
